# EUROPEAN PATENT APPLICATION

(11) **EP 4 063 486 A1**
(43) Date of publication of application: **28.09.2022**
(21) Application number: 20890313.8
(22) Date of filing: 17.11.2020
(51) Int. Cl.: C12N 5/071, C12Q 1/02

(54) **CELL STRUCTURE, PRODUCTION METHOD THEREOF, AND METHOD FOR EVALUATING HEPATOTOXICITY OF SUBSTANCE TO BE TESTED**

(30) Priority: 19.11.2019 JP 2019208933
(71) Applicant: TOPPAN INC., Tokyo 110-0016 (JP); OSAKA UNIVERSITY, Suita-shi Osaka 565-0871 (JP)
(72) Inventor: HIRAOKA Yasuyuki, Tokyo 110-0016 (JP); KITANO Shiro, Tokyo 110-0016 (JP); IRIE Shinji, Tokyo 110-0016 (JP); MATSUSAKI Michiya, Suita-shi, Osaka 565-0871 (JP)
(74) Representative: TBK
(86) International application number: PCT/JP2020/042824
(87) International publication number: WO 2021/100709

(57) **Abstract**

The present invention relates to a cell structure including cells containing at least hepatocytes and vascular endothelial cells, and extracellular matrix component, wherein the extracellular matrix components are disposed between the cells, and a liver sinusoidal network is provided between the cells.

## Description

### Technical Field

The present invention relates to a cell structure and a production method thereof, and a method for evaluating the hepatotoxicity of a substance to be tested.

### Background Art

Drug-induced liver injury (DILI) is a primary factor in drug development/discontinuation. Since the mechanisms of action of DILI are complicated and diverse, and there are quite a few toxicities expressed by human-specific biological reactions, it is not always easy to perform ex-ante prediction thereof in animal tests. Therefore, there is a need for liver tissues as an alternative tool for ex-ante prediction of such human-specific toxicity.

As a method for artificially producing a structure resembling a living tissue, for example, a method for producing a three-dimensional tissue by culturing coated cells in which the entire surface of cultured cells is covered with an adhesive membrane (Patent Literature 1), a method for producing a three-dimensional cell tissue including obtaining a mixture by mixing cells with a cationic substance and extracellular matrix components, collecting cells from the obtained mixture, and forming a cell aggregate on a substrate (Patent Literature 2) and the like are known. In addition, the inventors propose a method in which cells are brought into contact with endogenous collagen, and preferably, fibrous exogenous collagen is additionally brought into contact therewith, and thus a three-dimensional tissue having a high collagen concentration is produced (Patent Literature 3).

### Citation List

### Patent Literature

[Patent Literature 1] Japanese Unexamined Patent Publication No. 2012-115254
[Patent Literature 2] PCT International Publication No. WO2017/146124
[Patent Literature 3] PCT International Publication No. WO2018/143286

### Summary of Invention

### Technical Problem

In vitro liver tissue that can three-dimensionally reproduce a higher-order tissue morphology such as a vascular structure and enable accurate toxicity evaluation has not yet been constructed.

An object of the present invention is to provide a cell structure having better responsiveness to a substance having hepatotoxicity and a production method thereof.

### Solution to Problem

That is, the present invention relates to, for example, the following inventions.
[1] A cell structure, including
   cells containing at least hepatocytes and vascular endothelial cells, and extracellular matrix components,
   wherein the extracellular matrix components are disposed between the cells, and
   wherein a liver sinusoidal network is provided between the cells.
[2] The cell structure according to [1], wherein the proportion of the number of hepatocytes in the total number of cells is 60% or more and 80% or less.
[3] The cell structure according to [1] or [2], wherein the proportion of the number of vascular endothelial cells in the total number of cells is 5% or more and 35% or less.
[4] The cell structure according to any one of [1] to [3], wherein the extracellular matrix components are fibrous.
[5] The cell structure according to any one of [1] to [4], wherein the extracellular matrix components include collagen components.
[6] The cell structure according to any one of [1] to [5], containing a polyelectrolyte.
[7] The cell structure according to any one of [1] to [6], wherein the extracellular matrix components include fragmented extracellular matrix components.
[8] The cell structure according to any one of [1] to [7], wherein the vascular endothelial cells are sinusoidal endothelial cells.
[9] The cell structure according to any one of [1] to [8], wherein the cells further include hepatic stellate cells.
[10] A method for evaluating the hepatotoxicity of a substance to be tested, including:
   a culture step of culturing the cell structure according to any one of [1] to [9] in a state of being in contact with a substance to be tested;
   wherein the presence or absence or the degree of hepatotoxicity is evaluated using a cell viability, an amount of albumin produced, an adenosine triphosphate content, or having a liver sinusoidal network in the cell structure as an index after the culture step.
[11] The method for evaluating the hepatotoxicity of a substance to be tested according to [10], wherein the culture step is performed by culturing the cell structure in a medium containing the substance to be tested.
[12] A method for producing a cell structure, including:
   a contact step of contacting cells containing at least hepatocytes and vascular endothelial cells with extracellular matrix components in an aqueous medium; and
   a culture step of culturing the cells in contact with the extracellular matrix components,
   wherein the contact step is performed under conditions in which aggregation of the extracellular matrix components in an aqueous medium is restricted, and
   wherein the culture step is performed under conditions suitable for culturing hepatic non-parenchymal cells.
[13] The method for producing a cell structure according to [12], wherein the extracellular matrix components are fibrous.
[14] The method for producing a cell structure according to [12] or [13], wherein the contact step is performed by mixing the cells, the extracellular matrix components, and a polyelectrolyte.
[15] The method for producing a cell structure according to [14], wherein the polyelectrolyte is heparin.
[16] The method for producing a cell structure according to any one of [12] to [15], wherein the contact step includes accumulating the cells and the extracellular matrix components after the cells are brought into contact with the extracellular matrix components.
[17] The method for producing a cell structure according to any one of [12] to [16], wherein the extracellular matrix components include collagen components.
[18] The method for producing a cell structure according to any one of [12] to [17], wherein the extracellular matrix components include fragmented extracellular matrix components.
[19] The method for producing a cell structure according to [18], wherein the fragmented extracellular matrix components are defibrated extracellular matrix components.
[20] The method for producing a cell structure according to any one of [12] to [19], wherein the vascular endothelial cells are sinusoidal endothelial cells.
[21] The method for producing a cell structure according to any one of [12] to [20], wherein the cells further include hepatic stellate cells.
[22] The method for producing a cell structure according to any of [12] to [21], wherein the culture step is performed in the presence of angiogenesis promoting factors.

### Advantageous Effects of Invention

According to the present invention, it is possible to provide a cell structure having better responsiveness to a substance having hepatotoxicity and a production method thereof.

The cell structure of the present invention can maintain a liver function for a relatively long period (for example, 2 weeks or more). When the cell structure of the present invention is used, it is possible to evaluate the hepatotoxicity of a substance to be tested.

### Brief Description of Drawings

FIG. 1 shows images of the results of microscopic observation of a cell structure of Example 1 according to CD31 staining and albumin staining.
FIG. 2 shows images of the results of microscopic observation of a cell structure of Example 2 according to CD31 staining and albumin staining.
FIG. 3 shows images of the results of microscopic observation of a cell structure of Example 3 according to CD31 staining and albumin staining.
FIG. 4 shows images of the results of microscopic observation of a cell structure of Example 4 according to CD31 staining and albumin staining.
FIG. 5 shows images of the results of microscopic observation of a cell structure of Example 5 according to CD31 staining and albumin staining.
FIG. 6 shows images of the results of microscopic observation of a cell structure of Comparative Example 1 according to CD31 staining and albumin staining.
FIG. 7 shows images of the results of microscopic observation of a cell structure of Example 6 according to CD31 staining.
FIG. 8 shows images of the results of microscopic observation of a cell structure of Example 7 according to CD31 staining.
FIG. 9 shows images of the results of microscopic observation of a cell structure of Example 8 according to CD31 staining.
FIG. 10 is a graph showing the measurement results of an amount of albumin secreted.
FIG. 11 is a graph showing the evaluation results of responsiveness to Nefadazone.
FIG. 12 is a graph showing the evaluation results of responsiveness to Troglitazone.
FIG. 13 shows images of the results of a monocrotaline administration experiment.
FIG. 14 shows images of the results of a monocrotaline administration experiment, and enlarged images of FIG. 13.
FIG. 15 is a graph showing the analysis results of a liver sinusoidal network in a monocrotaline administration experiment.
FIG. 16 is a graph showing the results of ATP assay in a monocrotaline administration experiment.

### Description of Embodiments

Hereinafter, forms for implementing the present invention will be described in detail. However, the present invention is not limited to the following embodiments.

### [Cell structure]

A cell structure according to the present embodiment includes cells containing at least hepatocytes and vascular endothelial cells, and an extracellular matrix, and an extracellular matrix is disposed between the cells. The cell structure according to the present embodiment can be used as a liver tissue (liver model), which is a living tissue model having functions similar to at least some functions of the liver and/or having a structure similar to at least a part of a structure of the liver.

In this specification, the "cell structure" is a cell aggregate (mass-like cell population) in which cells are three-dimensionally disposed, and is an aggregate artificially produced by cell culture. Extracellular matrix components may be disposed between at least some cells. In the cell structure, a part in which cells are in direct contact with each other may be provided.

The shape of the cell structure is not particularly limited, and examples thereof include a sheet shape, a spherical shape, substantially a spherical shape, an ellipsoidal shape, substantially an ellipsoidal shape, a hemispherical shape, substantially a hemispherical shape, a semicircular shape, substantially a semicircular shape, a rectangular parallelepiped shape, and substantially a rectangular parallelepiped shape. Here, the living tissue includes sweat glands, lymphatic vessels, sebaceous glands, and the like, and has a more complex configuration than the cell structure. Therefore, the cell structure and the living tissue can be easily distinguished. In addition, the cell structure may be aggregated in a mass in which the cell structure is adhered to a support or aggregated in a mass in which the cell structure is not adhered to a support.

### (Cells)

The cells may be somatic cells or germ cells. In addition, the cells may be stem cells, or may be cultured cells such as primary cultured cells, sub-cultured cells and cell line cells. In this specification, the "stem cells" are cells having a self-replication ability and pluripotency. Stem cells include pluripotent stem cells having an ability to differentiate into any cell tumor and tissue stem cells (also called somatic stem cells) having an ability to differentiate into specific cell tumors. Examples of pluripotent stem cells include embryonic stem cells (ES cells), somatic cell-derived ES cells (ntES cells) and induced pluripotent stem cells (iPS cells). Examples of tissue stem cells include mesenchymal stem cells (for example, bone marrow-derived stem cells), hematopoietic stem cells and neural stem cells.

The total number of cells constituting the cell structure according to the present embodiment is not particularly limited, and is appropriately determined in consideration of the thickness and shape of the cell structure to be constructed, the size of the cell culture container used for construction, and the like.

In the cell structure according to the present embodiment, the cells include at least hepatocytes and vascular endothelial cells.

Hepatocytes are also referred to as hepatic parenchymal cells, and are, for example, cells having functions such as bile secretion and plasma protein secretion. The hepatocytes constituting the cell structure may be primary hepatocytes collected from the liver of an animal, cells obtained by culturing primary hepatocytes, cultured cell lines in which primary hepatocytes are established, or hepatoblasts artificially differentiated from stem cells. Examples of primary hepatocytes include primary human hepatocytes such as PXB Cell. Examples of cultured cell lines include cell lines derived from inactivated liver cancer cells such as HepG2. Examples of stem cells that differentiate into hepatoblasts include embryonic stem cells (ES cells), induced pluripotent stem cells (iPS cells), and mesenchymal stem cells. The hepatocytes contained in the cell structure according to the present embodiment are preferably non-cancerous cells such as primary hepatocytes and hepatoblasts, and more preferably PXB Cell in consideration of ease of handling.

The hepatocytes contained in the cell structure may be of one type or two or more types. For example, the cell structure may contain a plurality of hepatocytes having different genotypes for proteins involved in liver functions. In addition, conversely, all hepatocytes contained in the cell structure may have the same genotype for proteins involved in liver functions. Examples of proteins involved in liver functions include drug-metabolizing enzymes.

The proportion (X₁/X₀×100) of the number of hepatocytes (X₁) in the total number of cells (X₀) in the cell structure may be 5% or more, 10% or more, 15% or more, 20% or more, 25% or more, 30% or more, 35% or more, 40% or more, 45% or more, 50% or more, 55% or more, 60% or more, or 65% or more, and may be 95% or less, 90% or less, 80% or less or 75% or less. The proportion (X₁/X₀×100) of the number of hepatocytes (X₁) in the total number of cells (X₀) in the cell structure may be 60% or more and 80% or less, or 60% or more and 70% or less in order to obtain a more suitable liver tissue.

Vascular endothelial cells are squamous cells constituting the surface of the intravascular lumen. Vascular endothelial cells may be, for example, sinusoidal endothelial cells or human umbilical vein-derived vascular endothelial cells (HUVEC). The sinusoidal endothelial cells are hepatic non-parenchymal cells (cells other than hepatocytes among cells constituting the liver), and are cells which have a large number of small pore aggregates (cribriform plate structure) in the cytoplasm, and have a characteristic morphology different from other vascular endothelial cells such as lacking of a basement membrane. The vascular endothelial cells constituting the cell structure may be primary cells (primary vascular endothelial cells) collected from the liver of an animal (for example, human), cells in which primary cells are cultured, cultured cell lines in which primary cells are established, or cells artificially differentiated from stem cells. Examples of primary vascular endothelial cells include primary sinusoidal endothelial cells such as product model number 5000 (commercially available from Sciencell). Examples of cultured cell lines include cultured cell lines such as product model number T0056 (commercially available from Applied Biological Materials). Examples of differentiated stem cells include embryonic stem cells (ES cells) and induced pluripotent stem cells (iPS cells). The vascular endothelial cells contained in the cell structure according to the present embodiment may be non-cancerous cells.

The proportion (X₂/X₀×100) of the number of vascular endothelial cells (X₂) in the total number of cells (X₀) in the cell structure may be 5% or more, 10% or more, 12% or more, 14% or more, 15% or more, 20% or more, or 25% or more, and may be 50% or less, 45% or less, 40% or less, 35% or less, 30% or less, 25% or less, 20% or less, or 18% or less. The proportion (X₂/X₀×100) of the number of vascular endothelial cells (X₂) in the total number of cells (X₀) in the cell structure may be 5% or more and 40% or less, 5% or more and 35% or less, 10% or more and 35% or less, 10% or more and 25% or less, or may be 12% or more and 20% or less in order to obtain a more suitable liver tissue. The number of sinusoidal endothelial cells in the total number of cells in the cell structure may be within the above range.

In the cell structure according to the present embodiment, the cells may further include hepatic stellate cells in order to obtain a more suitable liver tissue. The hepatic stellate cells are hepatic non-parenchymal cells (cells other than hepatocytes among cells constituting the liver), and have a function of storing vitamin A, and are present in the space of Disse, which is an area between hepatocytes and the sinusoid in the liver. The hepatic stellate cells may be, for example, primary cells (primary hepatic stellate cells) collected from the liver of an animal (for example, a human), cells obtained by culturing primary cells, cultured cell lines in which primary cells are established, or cells artificially differentiated from stem cells. Examples of primary hepatic stellate cells include primary hepatic stellate cells such as model number 5300 (commercially available from Sciencell). Examples of cultured cell lines include cultured cell lines such as LX-2. Examples of differentiated stem cells include embryonic stem cells (ES cells), induced pluripotent stem cells (iPS cells), and mesenchymal stem cells. The hepatic stellate cells contained in the cell structure according to the present embodiment may be non-cancerous cells.

The proportion (X₃/X₀×100) of the number of hepatic stellate cells (X₃) in the total number of cells (X₀) in the cell structure may be 1% or more, 2% or more, 3% or more, 4% or more, or 5% or more, and may be 20% or less, 15% or less, 14% or less, 13% or less, 12% or less or 11% or less. The proportion (X₃/X₀×100) of the number of hepatic stellate cells (X₃) in the total number of cells (X₀) in the cell structure may be 1% or more and 15% or less, or 3% or more and 12% or less, in order to obtain a more suitable liver tissue.

In the present embodiment, the cells may include cells other than hepatocytes, vascular endothelial cells and hepatic stellate cells. The other cells may be, for example, matured somatic cells, or undifferentiated cells such as stem cells. Specific examples of somatic cells include for example, nerve cells, dendritic cells, immune cells, lymphatic endothelial cells, fibroblasts, epithelial cells (excluding hepatocytes), cardiomyocytes, pancreatic islet cells, smooth muscle cells, bone cells, alveolar epithelial cells, and spleen cells. Examples of stem cells include ES cells, iPS cells, and mesenchymal stem cells. The other cells may be normal cells or cells in which any cell function is enhanced or inhibited such as cancer cells. "Cancer cells" are cells derived from somatic cells and having an acquired infinite proliferative ability.

The cells in the cell structure may or may not contain mesenchymal stem cells as other cells. The proportion (X₄/X₀×100) of the number of mesenchymal stem cells (X₄) in the total number of cells (X₀) in the cell structure may be 5% or more, 10% or more, 15% or more, 20% or more, 25% or more, 30% or more, 35% or more, 40% or more, or 45% or more, and may be 80% or less, 70% or less, 60% or less, or 55% or less.

The origin of hepatocytes, vascular endothelial cells, and hepatic stellate cells contained in the cells or the other cells is not particularly limited, and for example, cells derived from mammals such as humans, monkeys, dogs, cats, rabbits, pigs, cows, mice, and rats may be used.

The cells in the cell structure according to the present embodiment may not include cells induced to differentiate from induced pluripotent stem cells (iPS cells). When the cells in the cell structure according to the present embodiment do not contain cells induced to differentiate from iPS cells, it is easier to determine a degree of differentiation from iPS cells, a proportion of differentiated cells in all cells, and the like, and as a result, it is easier to determine the content of each of the cells constituting the cell structure.

In the cell structure according to the present embodiment, vessels are formed between at least some cells. "Vessels are formed between cells" means that the tubular structure formed by vascular endothelial cells has a structure extending between cells and cells. The cell structure may have gaps in which no vessels are formed between cells. The gap may be an empty space, but may be filled with extracellular matrix components or the like.

It is said that it is difficult to maintain an artificially produced thick cell structure in the absence of vessels, and it is necessary to supply oxygen and the like from the outside. On the other hand, in the cell structure according to the present embodiment, since vessels are formed between cells like a living tissue, it is expected that the cell structure will be able to be maintained for a long period. In addition, it is also expected that it will be easier to engraft when transplanted to mammals and the like.

The cell structure according to the present embodiment preferably has a liver sinusoidal network between cells. "Having a liver sinusoidal network" means having a structure formed into a mesh such that a tubular structure formed by vascular endothelial cells branches and surrounds cells. The vascular structure between cells and the liver sinusoidal network can be confirmed by immunohistochemical staining. For example, the cell structure may have a liver sinusoidal network to the extent that it has a plurality of bifurcation points and is observed to have a mesh structure when observed from the upper surface under a microscope.

### (Extracellular matrix components)

The cell structure according to the present embodiment includes extracellular matrix components. The extracellular matrix components are disposed between at least some cells.

In this specification, the "extracellular matrix component" is an aggregate of extracellular matrix molecules formed by a plurality of extracellular matrix molecules. The extracellular matrix is a substance that is present outside cells in an organism. As the extracellular matrix, any substance can be used as long as it does not adversely affect cell growth and formation of cell aggregates. Specific examples include collagen, elastin, proteoglycan, fibronectin, hyaluronic acid, laminin, vitronectin, tenascin, entactin, fibrillin and cadherin, but the present invention is not limited thereto. The extracellular matrix components may be used alone or a combination thereof may be used. The extracellular matrix components may contain, for example, collagen components, or may be collagen components. When the extracellular matrix components are collagen components, the collagen component functions as a scaffold for cell adhesion, and formation of a three-dimensional cell structure is further promoted. The extracellular matrix component in the present embodiment is preferably a substance present outside cells of an animal, that is, an extracellular matrix component of an animal. Here, the extracellular matrix molecule may be a modifier or a variant of the above extracellular matrix molecule or may be a polypeptide such as a chemically synthesized peptide as long as it does not adversely affect cell growth and formation of cell aggregates.

The extracellular matrix components may have a repetition of a sequence represented by Gly-X-Y, which is a characteristic of collagen. Here, Gly represents a glycine residue, and X and Y each independently represent an arbitrary amino acid residue. The plurality of Gly-X-Y's may be the same as or different from each other. When a repetition of a sequence represented by Gly-X-Y is provided, there are few restrictions on the arrangement of molecular chains, and a function as a scaffolding material becomes better. In the extracellular matrix components having a repetition of a sequence represented by Gly-X-Y, the proportion of the sequence represented by Gly-X-Y among all amino acid sequences may be 80% or more and is preferably 95% or more. In addition, the extracellular matrix components may have an RGD sequence. The RGD sequence is a sequence represented by Arg-Gly-Asp (arginine residue - glycine residue - aspartic acid residue). When the extracellular matrix components have an RGD sequence, cell adhesion is further promoted, and the component becomes more suitable as a scaffolding material. The extracellular matrix components including the sequence represented by Gly-X-Y and the RGD sequence include collagen, fibronectin, vitronectin, laminin, cadherin and the like.

The shape of the extracellular matrix component is, for example, a fibrous shape. The fibrous shape is a shape composed of a filamentous extracellular matrix component or a shape composed of filamentous extracellular matrix components cross-linked between molecules. At least some of the extracellular matrix components may be fibrous. The shape of the extracellular matrix component is a shape of a mass of the extracellular matrix components (an aggregate of extracellular matrix components) when observed under a microscope, and the extracellular matrix components preferably have an average diameter and/or a size of an average length, which will be described below. The fibrous extracellular matrix components include fine filaments (fibrils) formed by aggregating a plurality of filamentous extracellular matrix molecules, filaments formed by additionally aggregating fibrils, and those obtained by defibrating these filaments. When the extracellular matrix components having a fibrous shape are contained, the RGD sequence is preserved without being destroyed in the fibrous extracellular matrix components, and the component can function as a scaffolding material for cell adhesion more effectively.

The extracellular matrix components may include fragmented extracellular matrix components. "Fragmented" means that an aggregate of extracellular matrix components is made smaller in size. The fragmented extracellular matrix components may include defibrated extracellular matrix components. The defibrated extracellular matrix component is a component obtained by defibrating the above extracellular matrix components by applying a physical force. For example, defibration is performed under conditions in which bonds in the extracellular matrix molecules are not broken.

The fragmented extracellular matrix components can be produced by, for example, a method including a step of fragmenting extracellular matrix components (fragmentation step).

A method for fragmenting extracellular matrix components is not particularly limited, and the component may be fragmented by applying a physical force. The extracellular matrix components fragmented by applying a physical force have generally the same molecular structure as that before fragmentation, unlike an enzymatic treatment (the molecular structure is maintained). A method for fragmenting extracellular matrix components may be, for example, a method for finely crushing mass extracellular matrix components. The extracellular matrix components may be fragmented in a solid phase or fragmented in an aqueous medium. For example, the extracellular matrix components may be fragmented by applying a physical force with an ultrasonic homogenizer, a stirring homogenizer, a high-pressure homogenizer or the like. When a stirring homogenizer is used, the extracellular matrix components may be directly homogenized, or may be homogenized in an aqueous medium such as a saline. In addition, it is possible to obtain fragmented extracellular matrix components having a millimeter size or a nanometer size by adjusting the homogenization time, the number of homogenizations, and the like. When the extracellular matrix components are fragmented in an aqueous medium, the fragmented extracellular matrix components can be produced, for example, by a method including a step in which extracellular matrix components are fragmented in an aqueous medium and a step in which an aqueous medium is removed from a liquid containing the fragmented extracellular matrix components and the aqueous medium (removal step). The removal step may be performed by, for example, a freeze-drying method. "Removing the aqueous medium" does not mean that no water is attached to the fragmented extracellular matrix components but means that there is less water than the extent that can be achieved by the above general drying method in common sense.

The diameter and length of the fragmented extracellular matrix components can be determined by analyzing individual fragmented extracellular matrix components under an electron microscope.

The average length of the fragmented extracellular matrix components may be 100 nm or more and 400 µm or less, or 100 nm or more and 200 µm or less. In one embodiment, the average length of the fragmented extracellular matrix components may be 5 µm or more and 400 µm or less, 10 µm or more and 400 µm or less, or 100 µm or more and 400 µm or less in order to easily form a thick cell structure. In another embodiment, the average length of the fragmented extracellular matrix components may be 100 µm or less, 50 µm or less, 30 µm or less, 15 µm or less, 10 µm or less, or 1 µm or less, and may be 100 nm or more. It is preferable that the average length of most of the fragmented extracellular matrix components among all the fragmented extracellular matrix components be within the above numerical value range. Specifically, preferably, the average length of 50% or more of the fragmented extracellular matrix components among all the fragmented extracellular matrix components is within the above numerical value range, and more preferably, the average length of 95% of the fragmented extracellular matrix components is within the above numerical value range. The fragmented extracellular matrix components are preferably fragmented collagen components having an average length within the above range.

The average diameter of the fragmented extracellular matrix components may be 50 nm to 30 µm, 4 µm to 30 µm, or 5 µm to 30 µm. The fragmented extracellular matrix components are preferably fragmented collagen components having an average diameter within the above range.

The average length and the average diameter of the fragmented extracellular matrix components can be determined by measuring individual fragmented extracellular matrix components under an optical microscope or the like and performing image analysis. In this specification, the "average length" is an average value of the measured lengths of samples in the longitudinal direction, and the "average diameter" is an average value of the measured lengths of samples in a direction orthogonal to the longitudinal direction.

The collagen components that are fragmented are also referred to as "fragmented collagen components." The "fragmented collagen components" are components which are obtained by fragmenting collagen components such as fibrous collagen components, and which maintain a triple helix structure. The average length of the fragmented collagen components is preferably 100 nm to 200 µm, more preferably 22 µm to 200 µm, and still more preferably 100 µm to 200 µm. The average diameter of the fragmented collagen components is preferably 50 nm to 30 µm, more preferably 4 µm to 30 µm, and still more preferably 20 µm to 30 µm.

At least some of the extracellular matrix components may be cross-linked between molecules and within molecules. The extracellular matrix components may be cross-linked within extracellular matrix molecules or between extracellular matrix molecules constituting the extracellular matrix components. When the extracellular matrix components include fragmented extracellular matrix components, at least some of the fragmented extracellular matrix components may be cross-linked between molecules or within molecules.

The extracellular matrix components of which at least some are cross-linked between molecules or within molecules can be produced by, for example, a method including a step of cross-linking extracellular matrix components (cross-linking step). The extracellular matrix components can include, for example, fragmented and cross-linked extracellular matrix components. The fragmented and cross-linked extracellular matrix components can be produced by, for example, a method including a step of fragmenting extracellular matrix components and a step of cross-linking the fragmented extracellular matrix components in that order, or a method including a step of cross-linking extracellular matrix components and a step of fragmenting the cross-linked extracellular matrix components in that order.

Examples of cross-linking methods include a physical cross-linking method by applying heat, ultraviolet rays, radiation, and the like, and a chemical cross-linking method using a cross-linking agent, an enzyme reaction and the like, and the method is not particularly limited. Physical cross-linking is preferable so that cell growth is not hindered. Cross-linking (physical cross-linking and chemical cross-linking) may be cross-linking via covalent bonds.

When the extracellular matrix components include collagen components, cross-links may be formed between collagen molecules (triple helix structure), and may be formed between collagen fibrils formed by collagen molecules. Cross-linking may be cross-linking by heat (thermal cross-linking). Thermal cross-linking can be performed, for example, by performing a heat treatment under a reduced pressure using a vacuum pump. When the collagen components are thermally cross-linked, the extracellular matrix components may be cross-linked when amino groups of collagen molecules form peptide bonds (-NH-CO-) with carboxyl groups of the same or other collagen molecules.

The extracellular matrix components can be cross-linked using a cross-linking agent. The cross-linking agent can, for example, cross-link carboxyl groups and amino groups, or can cross-link amino groups with each other. As the cross-linking agent, for example, an aldehyde-based, carbodiimide-based, epoxide-based or imidazole-based cross-linking agent is preferable in consideration of cost, safety and operability, and specific examples thereof include water-soluble carbodiimides such as glutaraldehyde, 1-ethyl-3-(3-dimethylaminopropyl)carbodiimide/hydrochloride, and 1-cyclohexyl-3-(2-morpholinyl-4-ethyl)carbodiimide/sulfonate.

The degree of cross-linking can be appropriately selected according to the type of the extracellular matrix components, a method for cross-linking, and the like. The degree of cross-linking may be 1% or more, 2% or more, 4% or more, 8% or more, or 12% or more, and may be 30% or less, 20% or less, or 15% or less. When the degree of cross-linking is within the above range, the extracellular matrix molecule can be appropriately dispersed, and the redispersibility after dry storage is favorable.

When amino groups in the extracellular matrix components are used for cross-linking, the degree of cross-linking can be quantified based on a TNBS (2,4,6-trinitrobenzenesulfonic acid) method described in Acta Biomaterialia, 2015, vol. 25, pp. 131-142. The degree of cross-linking by the TNBS method may be within the above range. The degree of cross-linking by the TNBS method is a proportion of amino groups used for cross-linking among amino groups of the extracellular matrix. When the extracellular matrix components include collagen components, the degree of cross-linking measured by the TNBS method is preferably within the above range.

The degree of cross-linking may be calculated by quantifying carboxyl groups. For example, in the case of extracellular matrix components that are insoluble in water, quantification may be performed by a TBO (toluidine blue O) method. The degree of cross-linking by the TBO method may be within the above range.

In the step of cross-linking, the temperature (heating temperature) and the time (heating time) when the extracellular matrix components are heated can be appropriately determined. The heating temperature may be, for example, 100°C or higher, 200°C or lower, or 220°C or lower. The heating temperature may be, specifically, for example, 100°C, 110°C, 120°C, 130°C, 140°C, 150°C, 160°C, 170°C, 180°C, 190°C, 200°C, or 220°C. The heating time (the time during which the heating temperature is maintained) can be appropriately set according to the heating temperature. When heating is performed at, for example, 100°C to 200°C, the heating time may be 6 hours or more and 72 hours or less, and is more preferably 24 hours or more and 48 hours or less. In the step of cross-linking, heating may be performed in the absence of a solvent or heating may be performed under a reduced pressure condition.

The content of the extracellular matrix components in the cell structure based on the dry weight of the cell structure may be 0.01 mass% or more, 0.05 mass% or more, 0.1 mass% or more, 0.5 mass% or more, 1 mass% or more, 2 mass% or more, 3 mass% or more, 4 mass% or more, 5 mass% or more, 6 mass% or more, 7 mass% or more, 8 mass% or more, 9 mass% or more, 10 mass%, 15 mass% or more, 20 mass% or more, 25 mass% or more, or 30 mass% or more, and may be 90 mass% or less, 80 mass% or less, 70 mass% or less, 60 mass% or less, 50 mass% or less, 30 mass% or less, 20 mass% or less, or 15 mass% or less. The content of the extracellular matrix components in the cell structure based on the dry weight of the cell structure may be 0.01 to 90 mass%, and may be 10 to 90 mass%, 10 to 80 mass%, 10 to 70 mass%, 10 to 60 mass%, 1 to 50 mass%, 10 to 50 mass%, 10 to 30 mass%, or 20 to 30 mass%.

Here, the "extracellular matrix components in the cell structure" are extracellular matrix components constituting the cell structure, and may be derived from endogenous extracellular matrix components or may be derived from exogenous extracellular matrix components.

The "endogenous extracellular matrix components" are extracellular matrix components produced from extracellular matrix-producing cells. Examples of extracellular matrix-producing cells include mesenchymal cells such as the above fibroblasts, chondrocytes, and osteoblasts. The endogenous extracellular matrix components may be fibrous or non-fibrous.

The "exogenous extracellular matrix components" are extracellular matrix components supplied from the outside. The cell structure according to the present embodiment contains fragmented extracellular matrix components, which are exogenous extracellular matrix components. The exogenous extracellular matrix components may be the same as or different from endogenous extracellular matrix components of the animal species from which they are derived. Examples of the originating animal species include humans, pigs, and cows. In addition, the exogenous extracellular matrix components may be artificial extracellular matrix components.

When the extracellular matrix components are collagen components, the exogenous extracellular matrix components are also referred to as "exogenous collagen components," and the "exogenous collagen components," which are collagen components supplied from the outside, are aggregates of collagen molecules formed by a plurality of collagen molecules, and specific examples thereof include fibrous collagen and non-fibrous collagen. The exogenous collagen component is preferably fibrous collagen. The fibrous collagen is a collagen component which is a main component of collagen fibers, and examples thereof include type I collagen, type II collagen, and type III collagen. As the fibrous collagen, commercially available collagen may be used, and specific examples thereof include pig-skin-derived type I collagen (commercially available from NH Foods Ltd.). Examples of exogenous non-fibrous collagen include type IV collagen.

In the exogenous extracellular matrix components, the originating animal species may be different from that of the cells. In addition, when the cells contain extracellular matrix-producing cells, in the exogenous extracellular matrix components, the originating animal species may be different from that of the extracellular matrix-producing cells. That is, the exogenous extracellular matrix components may be heterologous extracellular matrix components.

That is, when the cell structure contains endogenous extracellular matrix components and fragmented extracellular matrix components, the content of the extracellular matrix components constituting the cell structure is a total amount of the endogenous extracellular matrix components and the fragmented extracellular matrix components. The content of the extracellular matrix can be calculated from the volume of the obtained cell structure and the mass of the decellularized cell structure.

For example, when the extracellular matrix components contained in the cell structure are collagen components, as a method of quantifying the amount of the collagen components in the cell structure, for example, the following method of quantifying hydroxyproline may be exemplified. A sample is prepared by mixing hydrochloric acid (HCl) with a dissolution solution in which a cell structure is dissolved, incubating at a high temperature for a predetermined time, and then returning the temperature to room temperature, and diluting a centrifuged supernatant to a predetermined concentration. A hydroxyproline standard solution is treated in the same manner as the sample, and then gradually diluted to prepare a standard. Each of the sample and the standard is subjected to a predetermined treatment with a hydroxyproline assay buffer and a detection reagent, and the absorbance at 570 nm is measured. The amount of the collagen components is calculated by comparing the absorbance of the sample with that of the standard. Here, the cell structure may be directly suspended in high-concentration hydrochloric acid, and the dissolution solution in which the cell structure is dissolved may be centrifuged to recover the supernatant, which is used for quantifying the collagen components. In addition, the cell structure to be dissolved may be in a state in which it is recovered from the culture solution without change, or the cell structure may be dissolved when a drying treatment is performed after recovering and liquid components are removed. However, when the cell structure that is recovered from the culture solution is dissolved and the collagen components are quantified, since the measured value of the weight of the cell structure is expected to vary due to the influence of the medium component absorbed by the cell structure and the remaining medium due to the problem of the experimental technique, it is preferable to use the weight after drying as a reference in order to stably measure the weight of the tissue and the amount of the collagen components per unit weight.

As a method of quantifying the amount of the collagen components, more specifically, for example, the following method may be exemplified.

### (Preparation of sample)

A total amount of the frozen and dried cell structure is mixed with 6 mol/L HCl, and incubated at 95°C for 20 hours or more in a heat block, and the temperature is then returned to room temperature. After centrifuging at 13,000 g for 10 minutes, the supernatant of the sample solution is recovered. A sample is prepared by appropriately diluting with 6 mol/L HCl so that the result falls within a range of a calibration curve in the measurement to be described below and then performing dilution of 200 µL with 100 µL of ultrapure water. 35 µL of the sample is used.

### (Preparation of standard)

125 µL of a standard solution (1,200 µg/mL in acetic acid) and 125 µL of 12 mol/l HCl are put into a screw cap tube and mixed, and the mixture is incubated at 95°C for 20 hours in a heat block, and the temperature is then returned to room temperature. After centrifuging at 13,000 g for 10 minutes, the supernatant is diluted with ultrapure water to prepare 300 µg/mL of S1, and S1 is gradually diluted to prepare S2 (200 µg/mL), S3 (100 µg/mL), S4 (50 µg/mL), S5 (25 µg/mL), S6 (12.5 µg/mL), and S7 (6.25 µg/mL). S8 (0 µg/mL) containing only 90 µL of 4 mol/l HCl is also prepared.

### (Assay)

35 µL of the standard and the sample are put into plates (included in QuickZyme Total Collagen Assay kit, commercially available from QuickZyme Biosciences). 75 µL of an assay buffer (included in the above kit) is added to each well. The plate is closed with a seal and incubation is performed at room temperature while shaking for 20 minutes. The seal is removed, and 75 µL of a detection reagent (reagent A:B=30 µL: 45 µL, included in the above kit) is added to each well. The plate is closed with a seal, the solution is mixed with shaking, and incubation is performed at 60°C for 60 minutes. Cooling is sufficiently performed on ice, the seal is removed, and the absorbance at 570 nm is measured. The amount of the collagen components is calculated by comparing the absorbance of the sample with that of the standard.

The collagen components in the cell structure may be determined by an area ratio or volume ratio thereof. "Determined by the area ratio or the volume ratio" means that, for example, the collagen components in the cell structure are made distinguishable from other tissue structures by a known staining method (for example, immunostaining using anti-collagen antibodies or Masson trichrome staining), and the ratio of the existence area of the collagen components in the entire cell structure is then calculated using observation with the naked eye, various microscopes, image analysis software and the like. When the components are determined by the area ratio, the cross section or surface in the cell structure used in the determination according to the area ratio using, and for example, when the cell structure is a spherical component or the like, the components may be determined using a cross-sectional view that passes through substantially a center thereof.

For example, when the collagen components in the cell structure are determined by the area ratio, the ratio of the area based on the total area of the cell structure is generally 0.01 to 99%, and may be 1 to 99%, 5 to 90%, 7 to 90%, 20 to 90%, 30 to 90%, or 50 to 90%. The "collagen components in the cell structure" are as described above. The ratio of the area of the collagen components constituting the cell structure is a ratio of the area of the endogenous collagen components and the exogenous collagen components in combination. The obtained cell structure is stained with Masson trichrome, and the ratio of the area of the collagen components can be calculated, for example, as a ratio of the area of the collagen components stained with blue to the total area of the cross section that passes through substantially a center of the cell structure.

### (Polyelectrolyte)

A cell structure according to one embodiment may further contain a polyelectrolyte. The polyelectrolyte is a high-molecular-weight compound having properties of an electrolyte. Examples of polyelectrolytes include glycosaminoglycans such as heparin, chondroitin sulfate (for example, chondroitin 4-sulfate, chondroitin 6-sulfate), heparan sulfate, dermatan sulfate, keratan sulfate, and hyaluronic acid; dextran sulfate, rhamnan sulfate, fucoidan, carrageenan, polystyrene sulfonate, polyacrylamide-2-methylpropanesulfonic acid, and polyacrylic acid, or derivatives thereof, but the present invention is not limited thereto. The polyelectrolyte may be composed of one of the above examples, or may include two or more thereof in combination.

The polyelectrolyte is preferably glycosaminoglycan, more preferably contains at least one selected from the group consisting of heparin, dextran sulfate, chondroitin sulfate, and dermatan sulfate, and is still more preferably heparin. When the cell structure contains a polyelectrolyte, excessive aggregation of the extracellular matrix components can be reduced more effectively, and as a result, it is easier to obtain a cell structure having better responsiveness to a substance having hepatotoxicity. When the cell structure contains heparin, the effect becomes more significant.

The ratio (C₂/C₁) of the mass C₂ of the polyelectrolyte to the mass C₁ of the extracellular matrix components may be 1/100 to 100/1, 1/10 to 10/1, 1/5 to 5/1 or 1/2 to 2/1, and may be 1/1.5 to 1.5/1.

### (Cell structure)

The thickness of the cell structure may be 10 µm or more, 30 µm or more, 50 µm or more, 100 µm or more, 300 µm or more, or 1,000 µm or more. Such a cell structure is a structure closer to that of a living tissue, and is suitable as a substitute for an experimental animal and a material for transplantation. The upper limit of the thickness of the cell structure is not particularly limited, and may be, for example, 10 mm or less, 3 mm or less, 2 mm or less, 1.5 mm or less, or 1 mm or less.

Here, the "thickness of the cell structure" is a distance between both ends in a direction perpendicular to the main surface when the cell structure is sheet-like or a rectangular parallelepiped. When the main surface is uneven, the thickness is a distance at the thinnest part of the main surface.

When the cell structure is spherical or substantially spherical, the thickness of the cell structure is the diameter of the cell structure. When the cell structure is ellipsoidal or substantially ellipsoidal, the thickness of the cell structure is the breadth of the cell structure. When the cell structure is substantially spherical or substantially ellipsoidal, and the surface is uneven, the thickness of the cell structure is a distance between two points where the straight line passing through the center of gravity of the cell structure and the surface intersect, and is the shortest distance.

The cell structure preferably contains fragmented extracellular matrix components and/or a polyelectrolyte. In this case, the cell structure is more suitable as a tissue model for evaluating the hepatotoxicity or the like.

### (Fibrin)

The cell structure according to the present embodiment may contain fibrin. Fibrin is a component produced by the action of thrombin on fibrinogen to release Aα chains, A chains from the N terminal of Bβ chains, and B chains. Fibrin is a polymer and is generally insoluble in water. Fibrin is formed by bringing fibrinogen into contact with thrombin.

The cell structure according to the present embodiment is constructed in a cell culture container. The cell culture container is not particularly limited as long as it is a container in which a cell structure can be constructed and the constructed cell structure can be cultured. As the cell culture container, specifically, dishes, cell culture inserts (for example, Transwell (registered trademark) inserts, Netwell (registered trademark) inserts, Falcon (registered trademark) cell culture inserts, Millicell (registered trademark) cell culture inserts, etc.), tubes, flasks, bottles, plates, and the like may be exemplified. In the construction of the cell structure, dishes or various cell culture inserts are preferable so that it is possible to perform evaluation using the cell structure more appropriately.

### [Method for producing cell structure]

A method for producing a cell structure according to the present embodiment includes a contact step of contacting cells containing at least hepatocytes and vascular endothelial cells with extracellular matrix components in an aqueous medium and a culture step of culturing the cells in contact with the extracellular matrix components. Regarding the cells and the extracellular matrix components, those described above can be used. In the method for producing a cell structure according to the present embodiment, the contact step may be performed under conditions in which aggregation of the extracellular matrix components in the aqueous medium is restricted, and the culture step may be performed under conditions suitable for culturing hepatic non-parenchymal cells.

### (Contact step)

In the contact step, cells containing at least hepatocytes and vascular endothelial cells are brought into contact with extracellular matrix components in an aqueous medium. When cells are brought into contact with extracellular matrix components, the three-dimensional cell structure is expected to be easily obtained.

Bringing cells into contact with extracellular matrix components may be performed, for example, under conditions in aggregation of the extracellular matrix components in the aqueous medium is restricted. When cells are brought into contact with extracellular matrix components under the conditions, a liver sinusoidal network is more likely to be formed in the cell structure. In the cell structure, when cells contain hepatocytes, since hepatocytes tend to adhere to each other, the gap between the cells tends to be small, and as a result, it is thought that it is difficult to form a tubular structure such as a liver sinusoidal network. When cells are brought into contact with extracellular matrix components under conditions in which excessive aggregation of the extracellular matrix components in the aqueous medium is restricted, the distribution of the extracellular matrix components and the cells in the cell structure becomes more uniform, and as a result, it is speculated that a liver sinusoidal network is easily formed, and a cell structure having better responsiveness to a substance having hepatotoxicity is easily obtained.

Regarding the extracellular matrix components, for example, by providing the extracellular matrix components and a component restricting aggregation of the extracellular matrix components (for example, the above polyelectrolyte) together and/or by using fragmented extracellular matrix components as at least some of extracellular matrix components, it is possible to restrict aggregation in the aqueous medium. The condition in which aggregation of the extracellular matrix components in the aqueous medium is restricted may be, for example, a condition in which there is a polyelectrolyte and/or a condition including an extracellular matrix in which extracellular matrix components are fragmented.

The "aqueous medium" is a liquid containing water as an essential constituent. The aqueous medium may be, for example, an aqueous medium containing a cationic substance. The aqueous medium containing a cationic substance is, for example, a tris-hydrochloric acid buffer, a tris-maleic acid buffer, a bis-tris-buffer, or a cationic buffer such as HEPES (4-(2-hydroxyethyl)-1-piperazine ethanesulfonic acid), and the cationic substance may be a medium containing a cationic compound such as ethanolamine, diethanolamine, triethanolamine, polyvinylamine, polyallylamine, polylysine, polyhistidine, and polyarginine, and water. In addition, as the aqueous medium, a medium can be used. Examples of mediums include a liquid medium such as a Dulbecco's Modified Eagle medium (DMEM) and a vascular endothelial cell-dedicated medium (EGM2). The liquid medium may be a mixed medium in which two types of mediums are mixed.

The concentration and the pH of the cationic substance (for example, tris in a tris-hydrochloric acid buffer) in the aqueous medium containing a cationic substance are not particularly limited as long as it does not adversely affect cell growth and construction of the cell structure. For example, the concentration of the cationic substance based on the total amount of the aqueous medium containing a cationic substance may be 10 to 100 mM, or 40 to 70 mM, and may be 50 mM. The pH of the aqueous medium (for example, a cationic buffer) may be 6.0 to 8.0, 6.8 to 7.8, or 7.2 to 7.6.

For the contact step, methods such as a method in which an aqueous medium containing extracellular matrix components and a culture solution containing cells are mixed under the condition in which aggregation of the extracellular matrix components in the aqueous medium is restricted, a method in which an aqueous medium containing extracellular matrix components is added to a culture solution containing cells, a method in which cell are added to an aqueous medium containing extracellular matrix components, and a method in which extracellular matrix components and cells are added to an aqueous medium prepared in advance may be exemplified.

The order in which the cells are brought into contact with the extracellular matrix components is not particularly limited, and for example, some cells may be brought into contact with extracellular matrix components, the remaining cells may be then brought into contact with the extracellular matrix components, or all cells may be brought into contact with the extracellular matrix components simultaneously or substantially simultaneously.

An aqueous medium containing cells and an extracellular matrix may or may not be mixed by, for example, stirring, after each substance is added. The contact step may include incubating for a certain time after the cells are brought into contact with the extracellular matrix components.

The contact step may be performed after cells are accumulated in the aqueous medium. That is, the contact step may be performed by accumulating cells in the aqueous medium and then bringing them into contact with the extracellular matrix components. When the accumulated cells are brought into contact with the extracellular matrix components, it is easy to produce a cell structure having a high cell density in the bottom layer part. Cells may be accumulated by, for example, a method such as centrifugation and natural sedimentation.

In the contact step, the proportion (X₁/X₀×100) of the number of hepatocytes in the total number of cells (X₀) may be 5% or more, 10% or more, 15% or more, 20% or more, 25% or more, 30% or more, 35% or more, 40% or more, 45% or more, 50% or more, 55% or more, 60% or more, or 65% or more, and may be 95% or less, 90% or less, 80% or less or 75% or less. In the contact step, the proportion (X₁/X₀×100) of the number of hepatocytes (X₁) in the total number of cells (X₀) may be 60% or more and 80% or less, or 60% or more and 70% or less in order to obtain a more suitable liver tissue.

In the contact step, the proportion (X₂/X₀×100) of the number of vascular endothelial cells (X₂) in the total number of cells (X₀) may be 5% or more, 10% or more, 12% or more, 14% or more, 15% or more, 20% or more, or 25% or more, and may be 50% or less, 45% or less, 40% or less, 35% or less, 30% or less, 25% or less, 20% or less, or 18% or less. In order to obtain a more suitable liver tissue, the proportion (X₂/X₀×100) of the number of vascular endothelial cells (X₂) in the total number of cells (X₀) in the cell structure may be 5% or more and 40% or less, 5% or more and 35% or less, 10% or more and 35% or less, 10% or more and 25% or less, or 12% or more and 20% or less.

When the cells contain hepatic stellate cells, in the contact step, the proportion (X₃/X₀×100) of the number of hepatic stellate cells (X₃) in the total number of cells (X₀) may be 1% or more, 2% or more, 3% or more, 4% or more, or 5% or more, and may be 20% or less, 15% or less, 14% or less, 13% or less, 12% or less or 11% or less. In order to obtain a more suitable liver tissue, the proportion (X₃/X₀×100) of the number of hepatic stellate cells (X₃) in the total number of cells (X₀) in the cell structure may be 1% or more and 15% or less, or 3% or more and 12% or less.

In the contact step, the proportion (X₅/X₀×100) of the number of cells (X₅) other than hepatocytes, vascular endothelial cells and hepatic stellate cells in the total number of cells (X₀) may be 5% or more, 10% or more, 15% or more, 20% or more, 25% or more, 30% or more, 35% or more, 40% or more, or 45% or more, and may be 80% or less, 70% or less, 60% or less, or 55% or less. In the contact step, the proportion of the number of mesenchymal stem cells (X₄) in the total number of cells may be within the above range.

It is preferable that the cells not be derived from iPS cells. When cells not derived from iPS cells are used, it becomes easier to identify the type and content of cells in the cell structure, and as a result, it is more suitable as a tissue model for evaluating the hepatotoxicity.

In the contact step, the concentration of the extracellular matrix components can be appropriately determined according to the shape and thickness of a desired cell structure, the size of the incubator, and the like. For example, the concentration of the extracellular matrix components in the aqueous medium in the contact step may be 0.1 to 90 mass% or 1 to 30 mass%.

In the contact step, for example, the amount of the extracellular matrix components with respect to 1.0×10⁶ cells may be 0.1 to 100 mg, 0.5 to 50 mg, 0.8 to 25 mg, 1.0 to 10 mg, 1.0 to 5.0 mg, 1.0 to 2.0 mg, or 1.0 to 1.8 mg, may be 0.7 mg or more, 1.1 mg or more, 1.2 mg or more, 1.3 mg or more or 1.4 mg or more, or may be 7.0 mg or less, 3.0 mg or less, 2.3 mg or less, 1.8 mg or less, 1.7 mg or less, 1.6 mg or less or 1.5 mg or less. In the contact step, the amount of the fragmented extracellular matrix components may be within the above range.

In the contact step, the mass ratio (extracellular matrix component/cells) between the extracellular matrix components and the cells is preferably 1/1 to 1,000/1, more preferably 9/1 to 900/1, and still more preferably 10/1 to 500/1.

In a first embodiment, the contact step may be, for example, a step (a contact step A) in which the cells, extracellular matrix components, and a polyelectrolyte are mixed in an aqueous medium.

As the polyelectrolyte, those described above can be used. For example, the concentration of the polyelectrolyte with respect to a total amount of 1 mL of the aqueous medium may be more than 0 mg, 0.001 mg or more, 0.005 mg or more, 0.01 mg or more, 0.025 mg or more, 0.05 mg or more, or 0.075 mg or more, and may be less than 1.0 mg, 0.5 mg or less, or 0.1 mg or less.

A first contact step may be performed by, for example, mixing an extracellular-matrix-component-containing liquid containing extracellular matrix components and a first aqueous medium, a polyelectrolyte-containing liquid containing a polyelectrolyte and a second aqueous medium, and cells. In the extracellular-matrix-component-containing liquid, the extracellular matrix components may be dissolved or dispersed in the first aqueous medium. In the polyelectrolyte-containing liquid, the polyelectrolyte may be dissolved in the second aqueous medium. The first aqueous medium and the second aqueous medium may be the same type of aqueous medium or different types of aqueous mediums.

The order in which an extracellular-matrix-component-containing liquid, a polyelectrolyte-containing liquid and cells are mixed is not particularly limited, and mixing may be performed in any order. For example, a mixed solution in which an extracellular-matrix-component-containing liquid and a polyelectrolyte-containing liquid are mixed in advance may be prepared, and the mixed solution and cells may be mixed. In addition, all components may be substantially simultaneously mixed.

In the extracellular-matrix-component-containing liquid, the content of the extracellular matrix components with respect to the total amount of the extracellular-matrix-component-containing liquid may be 0.001 to 1.5 mg/mL, 0.05 to 1.5 mg/mL or 0.1 to 1.0 mg/mL.

In the polyelectrolyte-containing liquid, the content of the polyelectrolyte with respect to the total amount of the polyelectrolyte-containing liquid may be 0.001 to 10.0 mg/mL, 0.05 to 5.0 mg/mL, or 0.1 to 1.0 mg/mL.

In the contact step, the ratio (A₁:A₂) between the mass A₁ of the polyelectrolyte and the mass A₂ of the extracellular matrix components may be 1:200 to 200:1, 1:100 to 100:1, 1:10 to 10:1, or 1:5 to 5:1, may be 1:2 to 2:1, may be 1:1.5 to 1.5:1, or may be 1:1.

In a second embodiment, the contact step may be a step in which fragmented extracellular matrix components are brought into contact with cells (a contact step B). As the fragmented extracellular matrix components, those described above can be used. A second contact step may be performed using the fragmented extracellular matrix components in a part or all of the extracellular matrix components.

Incorporation of fibrinogen and/or thrombin may be provided in the contact step or after the contact step and before the culture step. For example, fibrinogen and thrombin may be added at the same time, or one may be added first and then the other may be added. In the contact step, for example, a first liquid containing extracellular matrix components, an aqueous medium and fibrinogen and a second liquid containing cells, an aqueous medium and thrombin may be mixed. When fibrinogen and/or thrombin is added, shrinkage that may occur in the culture step to be described below is more likely to be minimized, and the shape and size of the cell structure can be easily controlled. In addition, since a suspension containing cells and extracellular matrix components can be gelled, the state in which the cells and the extracellular matrix components are uniformly mixed is maintained, and the state in which the cells are brought close to the extracellular matrix components is easily maintained.

The contact step may include accumulation of cells and extracellular matrix components after the cells are brought into contact with the extracellular matrix components. When these components are accumulated, the distribution of extracellular matrix components and cell in the cell structure becomes more uniform. As a method of accumulating cells and extracellular matrix components, for example, a method of centrifuging a culture solution containing extracellular matrix components and cells, and a method of performing natural sedimentation may be exemplified.

### (Culture step)

In the culture step, cells in contact with extracellular matrix components are cultured. The cells in contact with extracellular matrices are cultured under conditions suitable for culturing hepatic non-parenchymal cells. The conditions suitable for culturing hepatic non-parenchymal cells are conditions in which cells other than hepatocytes (for example, sinusoidal endothelial cells) are more likely to grow than hepatocytes. The culture step may be performed by, for example, culturing the cells in a medium containing a medium for hepatic non-parenchymal cells, or may be performed by culturing the cells in a medium that does not contain a medium for hepatocytes and contains a medium for hepatic non-parenchymal cells.

The medium used in the culture step may not contain insulin and transferrin, which are proteins secreted from the liver. Examples of mediums used in the culture step include a medium for vascular endothelia (for example, EGM2 (commercially available from Lonza), EGM2-MV (commercially available from Lonza), Endothelial Cell Growth Medium 2 (commercially available from Promocell), Endothelial Cell Growth Medium MV 2 (commercially available from Promocell), and ECM (commercially available from Sciencell). The medium may be a medium to which serum is added or a serum-free medium. The medium may be a mixed medium in which two types of mediums are mixed. For example, a mixed medium in which a medium for vascular endothelia and a medium for mesenchymal stem cell proliferation are mixed may be used.

The culture step may be performed in the presence of angiogenesis promoting factors. As a medium in which the cells are cultured, a medium containing angiogenesis promoting factors may be used. Examples of angiogenesis promoting factors include a vascular endothelial growth factor (VEGF) and a fibroblast growth factor (FGF).

The culture temperature in the culture step may be, for example, 20°C to 40°C, or 30°C to 37°C. The pH of the medium may be 6 to 8, or 7.2 to 7.4. The culture time may be 1 day to 2 weeks, or 1 week to 2 weeks.

The incubator (support) is not particularly limited, and may be, for example, a dish, a well insert, a low adhesive plate, or a plate having a bottom shape such as a U shape, and a V shape. The cells may be cultured while adhered to the support, or the cells may be cultured without being adhered to the support or may be separated from the support during culture and cultured. When the cells are cultured without being adhered to the support or are separated from the support during culture and cultured, it is preferable to use a plate having a bottom shape such as a U shape and a V shape that inhibits adhesion of the cells to the support or a low adsorption plate.

The cell density in the medium in the culture step can be appropriately determined according to the shape and thickness of a desired cell structure, the size of the incubator, and the like. For example, the cell density in the medium in the culture step may be 1 to 10⁸ cells/mL or 10³ to 10⁷ cells/mL. In addition, the cell density in the medium in the culture step may be the same as the cell density in the aqueous medium in the contact step.

After the culture step (hereinafter referred to as a "first culture step," and an initial contact step is referred to as a "first contact step"), additionally, a step of contacting cells (a second contact step), and a step of culturing cells (a second culture step) may be provided. The cells in the second contact step and the second culture step may be the same species as or a different species from the cells used in the first contact step and the first culture step. According to the second contact step and the second culture step, it is possible to produce a cell structure having a two-layer structure. In addition, when the contact step and the culture step are repeatedly included, it is possible to produce a cell structure having a plurality of layers, and it is possible to produce a tissue that is closer to a more complex living body.

According to the production method of the present embodiment, it is possible to suitably produce a cell structure in which vessels are formed between cells and a cell structure having a liver sinusoidal network between cells.

### [Application of cell structure]

A cell structure according to the present embodiment can be applied as a substitute for an experimental animal, a material for transplantation and the like, and as specific examples, the cell structure can be applied for tissue reconstruction, pathological in vitro model, drug screening (drug evaluation), assay screening of cosmetics, and the like.

### [Method for evaluating hepatotoxicity of substance to be tested]

Since the cell structure according to the present embodiment has better responsiveness to a substance having hepatotoxicity, it can be suitably used as a tool for evaluating the presence or absence or the degree of hepatotoxicity of the substance to be tested. When the cell structure according to the present embodiment is used, it becomes easier to evaluate the hepatotoxicity of the substance to be tested with higher reliability. When the method for evaluating the hepatotoxicity of a substance to be tested using the cell structure according to the present embodiment, it is possible to perform screening of the substance having hepatotoxicity.

The method for evaluating the hepatotoxicity of a substance to be tested according to the present embodiment includes a culture step in which the above cell structure that is in contact with a substance to be tested is cultured. The hepatotoxicity evaluation method is not particularly limited, and for example, other assay methods (an MTT assay, an MTS assay, etc.) in which values that correlate with the cell viability, the ATP content, the amount of albumin produced, and the number of living cells of the cell structure are obtained, methods such as quantification of the amount of bile acid uptaken by hepatocytes and quantification of glutathione, or use of markers used in clinical evaluation of the presence or absence of hepatic disorders such as ALT and bilirubin, can be appropriately selected according to the mechanism of toxicity of interest.

In the method for evaluating the hepatotoxicity of a substance to be tested according to the present embodiment, for example, the presence or absence or the degree of hepatotoxicity may be evaluated using the cell viability, the amount of albumin produced, or the adenosine triphosphate (ATP) content of the cell structure as an index after the culture step.

In the evaluation step, the presence or absence or the degree of hepatotoxicity may be evaluated using the liver sinusoidal network (vascular network) of the cell structure as an index after the culture step. When the liver sinusoidal network is used as an index, specifically, for example, the degree of fragmentation of the liver sinusoidal network and/or the total vessel length of the liver sinusoidal network may be used as an index.

In the culture step, the cell structure can be brought into contact with the substance to be tested by, for example, adding a substance to be tested to the culture medium of the cell structure.

The substance to be tested may be, for example, a drug suspected of having hepatotoxicity.

The substance to be tested which is a target to be evaluated may be of one type or two or more types. When two or more types of compounds are evaluated as the substance to be tested, each compound that is brought into contact with the cell structure may be evaluated or a plurality of compounds that are brought into contact with the cell structure may be simultaneously evaluated.

The culture step may be performed when the cell structure is cultured in a medium containing a substance to be tested. The time for which the cell structure is cultured in the medium containing a substance to be tested is not particularly limited, and may be, for example, 24 to 96 hours, 48 to 96 hours, or 48 to 72 hours. As necessary, hydrodynamic addition such as reflux can be provided as long as the culture environment is not significantly changed.

A method for evaluating the presence or absence or the degree of hepatotoxicity using the cell viability of the cell structure as an index after the culture step, can be performed by, for example, the following method.

As compared with the case in which the cell structure is cultured in the absence of the substance to be tested, when the number of living hepatocytes in the cell structure is small (low survival rate), the substance to be tested is evaluated to be toxic to hepatocytes contained in the cell structure, that is, hepatotoxic. As compared with the case in which the substance to be tested is absent, when the degree of decrease in the hepatocyte survival rate is larger, the hepatotoxicity can be evaluated to be stronger. On the other hand, as compared with the case in which culture is performed in the absence of the substance to be tested, when the number of living hepatocytes is the same or significantly larger (the survival rate is the same or higher), the substance to be tested is evaluated to be non-hepatotoxic.

The number of living hepatocytes can be evaluated using a signal that correlates with living hepatocytes or its abundance. It suffices if the number of living hepatocytes can be measured during evaluation, and it is not always necessary to measure the number of cells in a living state. For example, hepatocytes can be labeled in order to distinguish them from other cells, and a signal from the label can be used as an index for examination. For example, after hepatocytes are fluorescently labeled, when it is determined whether cells are alive or dead, the number of living hepatocytes in the cell structure can be directly counted. In this case, an image analysis technique can also be used. Determination of whether cells are alive or dead can be performed by a known cell alive/dead determination method such as trypan blue staining and propidium iodide (PI) staining. Here, for example, fluorescently labeling hepatocytes can be performed by a known method such as an immunostaining method using antibodies for substances specifically expressed on the cell surface of hepatocytes as primary antibodies, and fluorescently labeled secondary antibodies that specifically bind to the primary antibodies. Determination of whether cells are alive or dead and measurement of the number of living cells may be performed in the state of the cell structure or may be performed when the cell structure is destroyed at the single cell level. For example, after the three-dimensional structure of the cell structure after hepatocytes and dead cells are labeled is destroyed, it is also possible to directly count the number of hepatocytes that are alive at the time of evaluation by fluorescence activated cell sorting (FACS) using the label as an index.

When hepatocytes in a living state in the cell structure are labeled, and a signal from the label is detected over time, it is also possible to measure the number of living hepatocytes in the cell structure over time. After the cell structure is constructed, hepatocytes in the cell structure may be labeled, or before the cell structure is constructed, hepatocytes may be labeled in advance. In addition, when hepatocytes that constantly express a fluorescent dye, the number of living hepatocytes can be evaluated by measuring a fluorescence intensity of a lysate obtained by dissolving the cell structure with a microplate reader or the like.

A method for evaluating the presence or absence or the degree of hepatotoxicity using the amount of albumin produced of the cell structure as an index after the culture step can be performed by, for example, the following method.

As compared with the case in which culture is performed in the absence of the substance to be tested, when the amount of albumin produced in the cell structure is small (the albumin-producing ability is low) or the ATP content in the cell structure is low, the substance to be tested at the concentration is evaluated to be toxic to hepatocytes contained in the cell structure, that is, hepatotoxic. As compared with the case in which the substance to be tested is absent, when the degree of decrease in the amount of albumin produced or the degree of decrease in the ATP content is larger, the hepatotoxicity can be evaluated to be stronger. On the other hand, as compared with the case in which culture is performed in the absence of the substance to be tested, when the amount of albumin produced is the same or significantly larger (the albumin-producing ability is the same or larger) or when the ATP content is the same or significantly larger, the substance to be tested at the concentration is evaluated to be non-hepatotoxic.

The amount of albumin produced can be evaluated by, for example, measuring albumin in the culture supernatant using an ELISA method.

Hepatocytes contained in the cell structure preferably have common genotypes of at least one or more types of drug-metabolizing enzymes and more preferably have common genotypes of all drug-metabolizing enzymes. Since the hepatotoxicity tends to depend on drug-metabolizing enzymes, when a cell structure containing hepatocytes having homologous drug-metabolizing enzyme genotypes is used, the hepatotoxicity can be evaluated more accurately.

A method for evaluating the presence or absence or the degree of hepatotoxicity using a liver sinusoidal network in the cell structure as an index after the culture step can be performed by, for example, a method including a step of quantifying a total vessel length of the liver sinusoidal network in the cell structure after the culture step. As a method of quantifying the total vessel length of the liver sinusoidal network, for example, the method described in examples to be described below can be performed. In the method for evaluating the hepatotoxicity of a substance to be tested according to the present embodiment, since the hepatotoxicity can be evaluated using the liver sinusoidal network in the cell structure as an index, it is possible to evaluate the presence or absence and the degree of hepatotoxicity of the substance to be tested with higher sensitivity. The method for evaluating the hepatotoxicity of a substance to be tested according to the present embodiment is particularly effective when the substance to be tested that is presumed to affect the liver sinusoidal network is evaluated.

### Examples

The present invention will be described below in more detail with reference to examples. However, the present invention is not limited to these examples.

In order to produce a cell structure, the following cells and mediums were prepared.

### (Cells)

Human hepatocytes: PXB cells, commercially available from PhoenixBio Co., Ltd.

Human mesenchymal stem cells (MSC): product model number PT-2501, commercially available from Lonza

Liver sinusoidal endothelial cells (SEC): product model number 5000, commercially available from Sciencell

Hepatic stellate cells (Lx2): product model number SCC064, commercially available from Merck Millipor

### (Medium)

Medium for mesenchymal stem cell proliferation: product name MSCGM2, product code C28009, commercially available from Promocell

Medium for vascular endothelia 1: product name ECM, product code 1001, commercially available from Sciencell

Medium for vascular endothelia 2: product name EGM2-MV, product code CC-3202, commercially available from Lonza

Medium for hepatocytes: product name d-HCGM, product code PPC-M200, commercially available from PhoenixBio Co., Ltd.

### <Test Example 1: Production of cell structure 1>

Heparin (commercially available from Sigma) was dissolved in a 20 mM tris-hydrochloric acid buffer (pH 7.4) to obtain a heparin solution. Based on the total mass of the heparin solution, the content of heparin was 1.0 mg/mL. Collagen (type I collagen, commercially available from Nippi. Inc.) was dissolved in a 5 mM acetic acid solution to obtain a collagen solution. Based on the total mass of the collagen solution, the content of collagen was 0.4 mg/mL.

A mixed solution in which 100 µL of the heparin solution and 100 µL of the collagen solution were mixed was PXB cells, SEC and Lx2, and as necessary, MSC were put into a microtube and suspended. The cell numbers of various cells were adjusted to the ratio shown in Table 1. The total number of cells per well was about 100,000 cells.

The obtained suspension was centrifuged under conditions of 25°C, 400×g for 1 minute. Thereby, a viscous component in which collagen and heparin were attached to the cell surface was formed. After stretching, the supernatant was removed, 250 µL of a mixed medium (mass ratio 1:1) containing a medium for vascular endothelia 1 (ECM) and a medium for mesenchymal stem cell proliferation (MSCGM2) was put into a microtube, and the obtained suspension was seeded in a 96-well cell culture insert (Roche Inc, E-Platelnsert 16). After the seeding, culture was performed in a CO₂ incubator (37°C, 5% CO₂) for a predetermined time to obtain a cell structure.

**[Table 1]**

| | Number of cells | | | |
|---|---|---|---|---|
| | PXB cell | MSC | SEC | Lx2 |
| Example 1 | 50% | 35% | 10% | 5% |
| Example 2 | 65% | None | 30% | 5% |
| Example 3 | 75% | None | 20% | 5% |

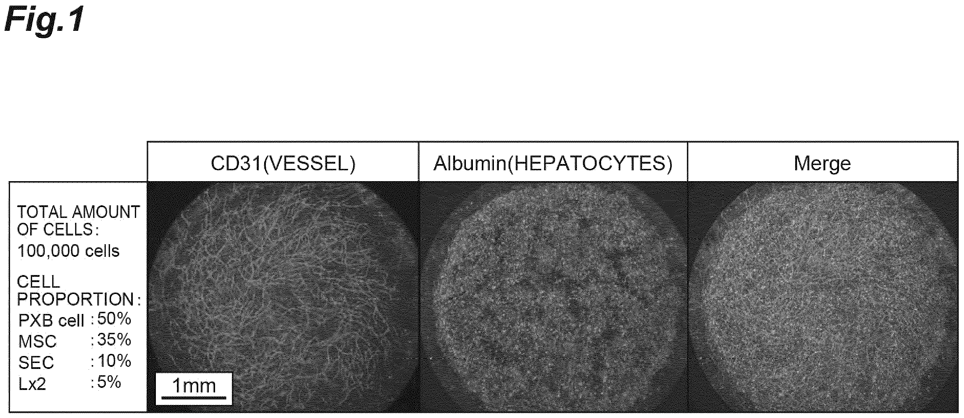

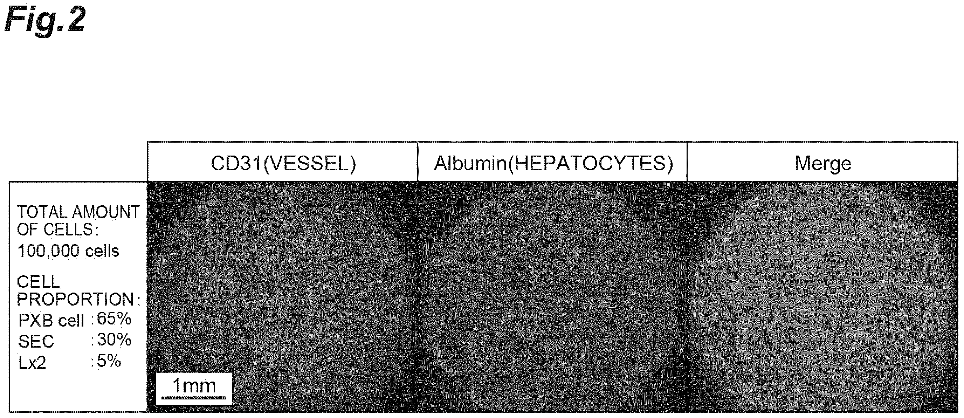

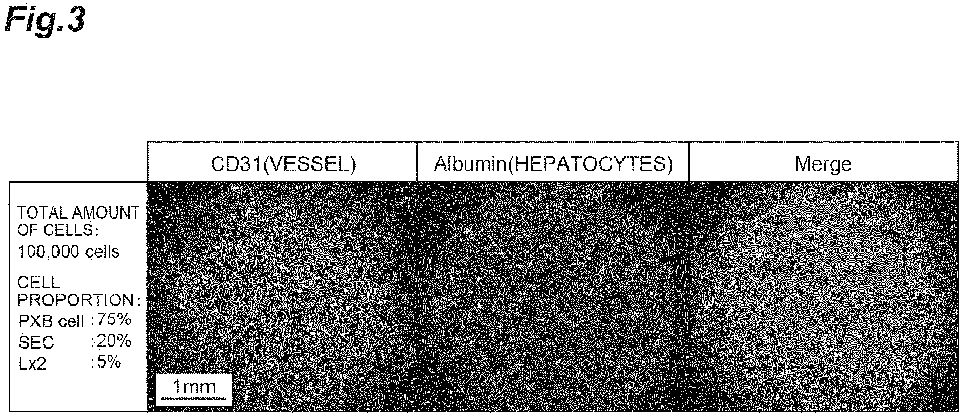

FIGS. 1 to 3 show images of the observation results of the cell structures of Examples 1 to 3. FIG. 1 shows the observation results of the sample of Example 1 immunostained with CD31 and albumin after being fixed on the 7^{th} day after the start day of culture. FIG. 2 shows the observation results of the sample of Example 2 immunostained with CD31 and albumin after being fixed on the 8^{th} day after the start day of culture. FIG. 3 shows the observation results of the sample of the example immunostained with CD31 and albumin after being fixed on the 8^{th} day after the start day of culture. The cell structures shown in FIGS. 1 to 3 had a liver sinusoidal network.

### <Test Example 2: Production of cell structure 2>

### [Production of defibrated collagen components]

100 mg of a type I collagen sponge fragment derived from pig skin (commercially available from NH Foods Ltd.) was heated at 200°C for 24 hours, and thus a collagen component of which at least a part was cross-linked (cross-linked collagen component) was obtained. Here, before and after heating at 200°C, no significant change in appearance was observed in the collagen. 50 mg of the cross-linked collagen component was put into a 15 mL tube, 5 mL of ultrapure water was added thereto, and the mixture was homogenized using a homogenizer (VH-10 commercially available from As One Corporation) for 6 minutes.

An aqueous solution containing the homogenized cross-linked collagen component was centrifuged at 10,000 rpm for 10 minutes under conditions of 21°C. The supernatant was suctioned, and collagen pellets were mixed with 5 mL of fresh ultrapure water to prepare a collagen solution. While the tube containing the collagen solution was kept on ice, an ultrasonic treatment was performed using a sonicator (VC50 commercially available from Sonics and Materials) at 100 V for 20 seconds, the sonicator was removed, and the tube containing the collagen solution was then cooled on ice for 10 seconds, which was repeated 100 times. After the ultrasonic treatment was performed 100 times, the collagen solution was filtered with a filter having a pore size of 40 µm to obtain a dispersion solution containing a defibrated collagen component (sCMF). The dispersion solution was freeze-dried by a conventional method to obtain a defibrated collagen component (sCMF) as a dried product. The average length (length) of sCMF was 14.8±8.2 µm (N=20).

### [Production of cell structure]

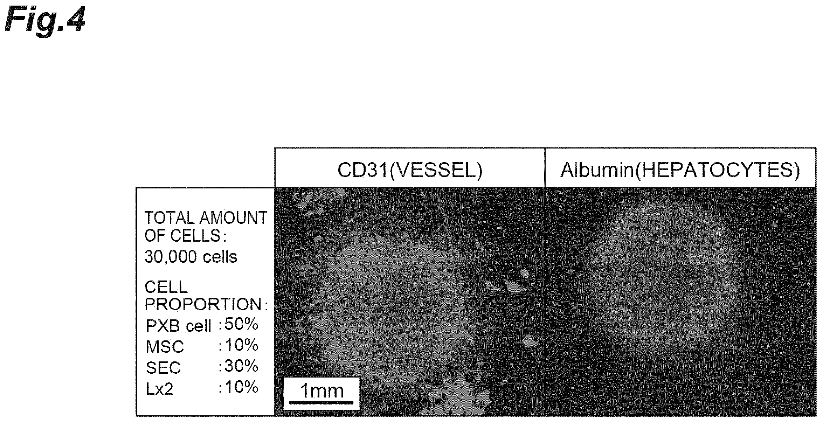

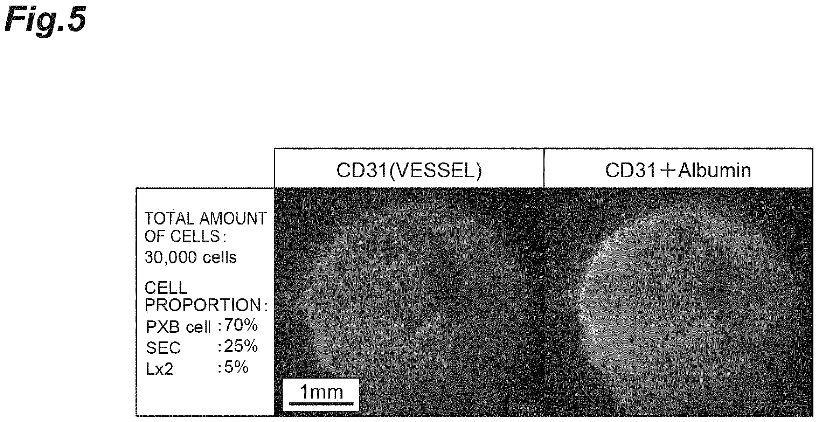

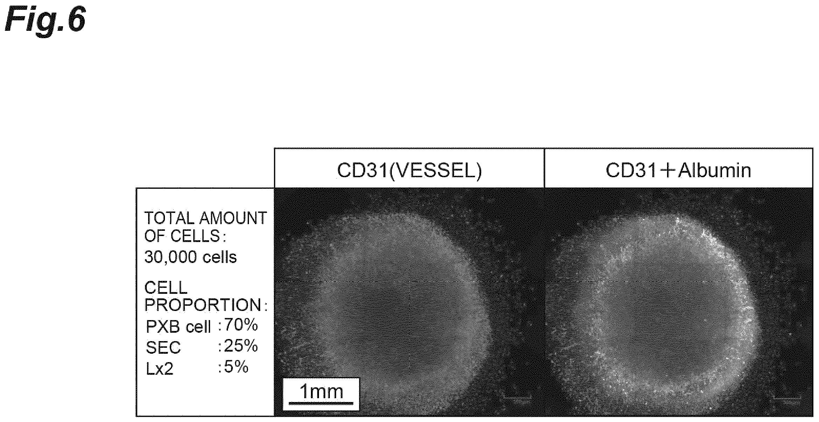

A dispersion solution A in which the defibrated collagen component was dispersed in a medium (DMEM) containing serum in which 10 mg/mL of fibrinogen (commercially available from Sigma) was dissolved so that the concentration was 30 mg/mL was obtained. In addition, a dispersion solution B in which cells were dispersed in a medium (DMEM) containing serum in which 10 U/mL of thrombin (commercially available from Sigma) was dissolved so that the total number of cells per well was 30,000 cells. 10 µL of a suspension in which the obtained dispersion solution A (defibrated collagen component) and dispersion solution B were mixed at 1:1 was added to each well of a 48-well plate. FIGS. 4 to 6 show the observation results of the culture product containing cells and defibrated collagen components 6 days after the start of the culture.

Regarding the medium, a mixed medium (mass ratio: 1:1) containing a medium for vascular endothelia 1 (ECM) and a medium for mesenchymal stem cell proliferation (MSCGM2), a medium for vascular endothelia 1 (ECM), or a medium for vascular endothelia 1 (ECM) and a medium for hepatocytes (d-HCGM) were used.

**[Table 2]**

| | Number of cells | | | | Medium |
|---|---|---|---|---|---|
| | PXB cell | MSC | SEC | Lx2 | |
| Example 4 | 50% | 10% | 30% | 10% | ECM+MSCGM2 |
| Example 5 | 70% | None | 25% | 5% | ECM |
| Comparative Example 1 | 70% | None | 25% | 5% | ECM+d-HCGM |

When a medium suitable for hepatic non-parenchymal cells was used, it was confirmed that a sinusoid network was formed in the cell structure (Examples 4 and 5). On the other hand, when a medium for hepatocytes was used, no liver sinusoidal network was formed in the obtained cell structure (Comparative Example 1).

### <Test Example 3: Production of cell structure 3>

A mixed solution in which 100 µL of the heparin solution and 100 µL of the collagen solution were mixed was PXB cells, SEC and Lx2, and as necessary, MSC were put into a microtube and suspended. The cell numbers of various cells were adjusted to the ratio shown in Table 3. The total number of cells was about 30,000 cells.

The obtained suspension was centrifuged under conditions of 25°C, 400×g for 1 minute. Thereby, a viscous component in which collagen and heparin were attached to the cell surface was formed. After stretching, the supernatant was removed, 250 µL of a mixed medium (mass ratio 1:1) containing a medium for vascular endothelia 1 (ECM) and a medium for mesenchymal stem cell proliferation (MSCGM2) was put into a microtube, and the obtained suspension was seeded in a 96-well cell culture insert (Roche Inc, E-Platelnsert 16). After the seeding, culture was performed in a CO₂ incubator (37°C, 5% CO₂) for a predetermined time to obtain a cell structure.

**[Table 3]**

| | Number of cells | | | |
|---|---|---|---|---|
| | PXB cell | MSC | SEC | Lx2 |
| Example 6 | 10% | 50% | 30% | 10% |
| Example 7 | 30% | 30% | 30% | 10% |

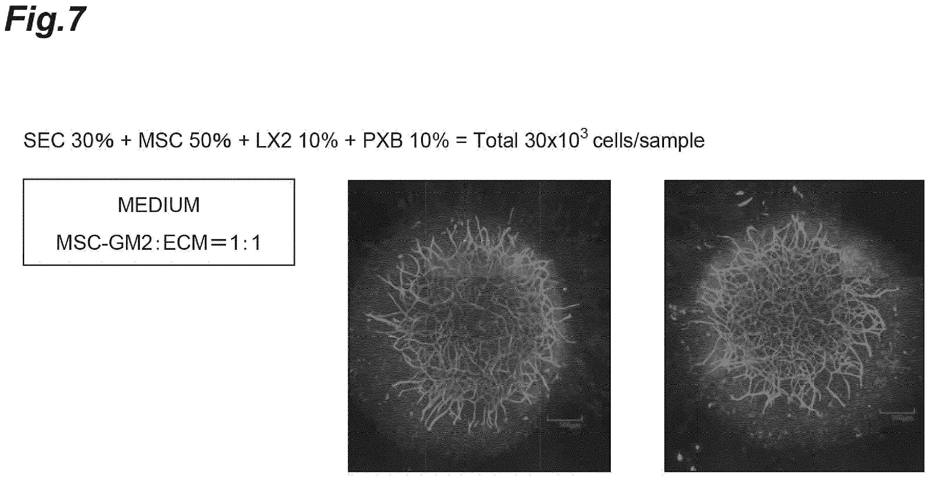

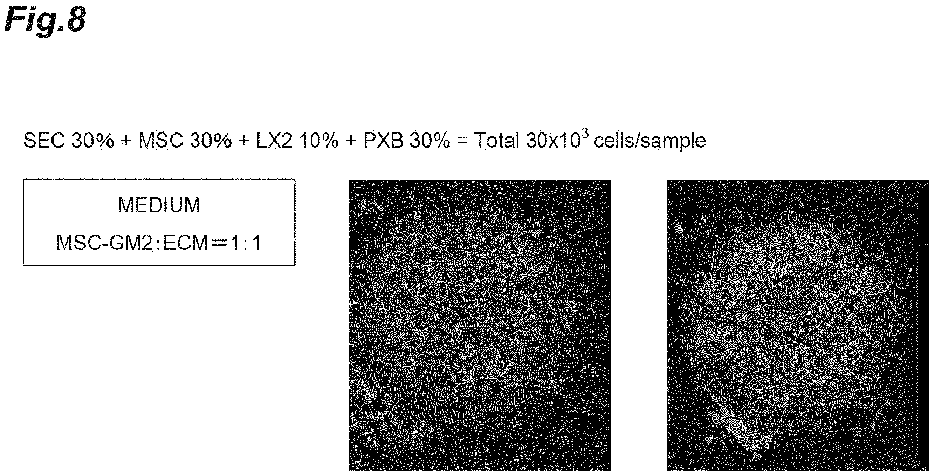

As shown in FIGS. 7 and 8, it was confirmed that a liver sinusoidal network was formed in the obtained cell structure even if the proportions of the numbers of cells was different from those of Examples 1 to 5.

### <Test Example 4: Production of cell structure 4>

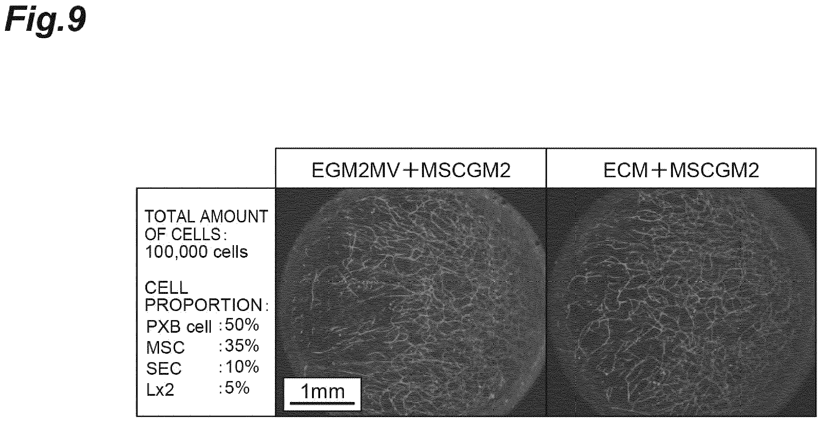

The cell structure of Example 8 was produced in the same manner as in Condition 1 of Test Example 1 except that the medium was changed to a medium for vascular endothelia 2 (EGM2MV), and the culture period was fixed on the 7^{th} day. The results are shown in FIG. 9. It was confirmed that a liver sinusoidal network was formed in the cell structure obtained using a different medium.

### <Test Example 5: Measurement of amount of albumin secreted>

In the cell structure produced by the method of Example 1, the amount of albumin secreted was measured by quantifying the amount of albumin in the culture supernatant during the culture period by ELISA. The medium was replaced on the day before the supernatant was recovered, and the culture supernatant of 24 hours was used as a sample for ELISA measurement. As a negative control, a cell structure produced in the same manner as in Example 1 except that no PXB Cells were contained was prepared.

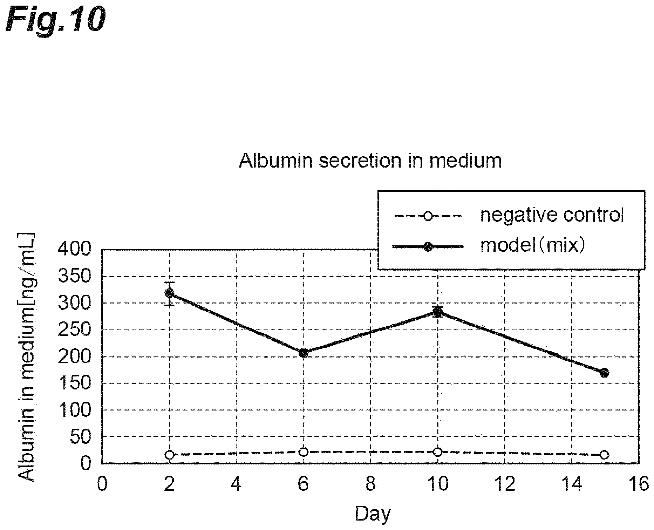

FIG. 10 shows the measurement results of the amount of albumin secreted. 15 days after the start of the culture, it was confirmed that significant albumin secretion was maintained for the negative control.

### <Test Example 6: Evaluation of hepatotoxicity>

In the hepatotoxicity evaluation test, the cell structures produced by the methods of Example 5 and Comparative Example 1 and 7 days after the start of the culture were used as cell structures for evaluation. For evaluation of the hepatotoxicity, Nefazodone and Troglitazone, which are drugs known to have hepatotoxicity, were used.

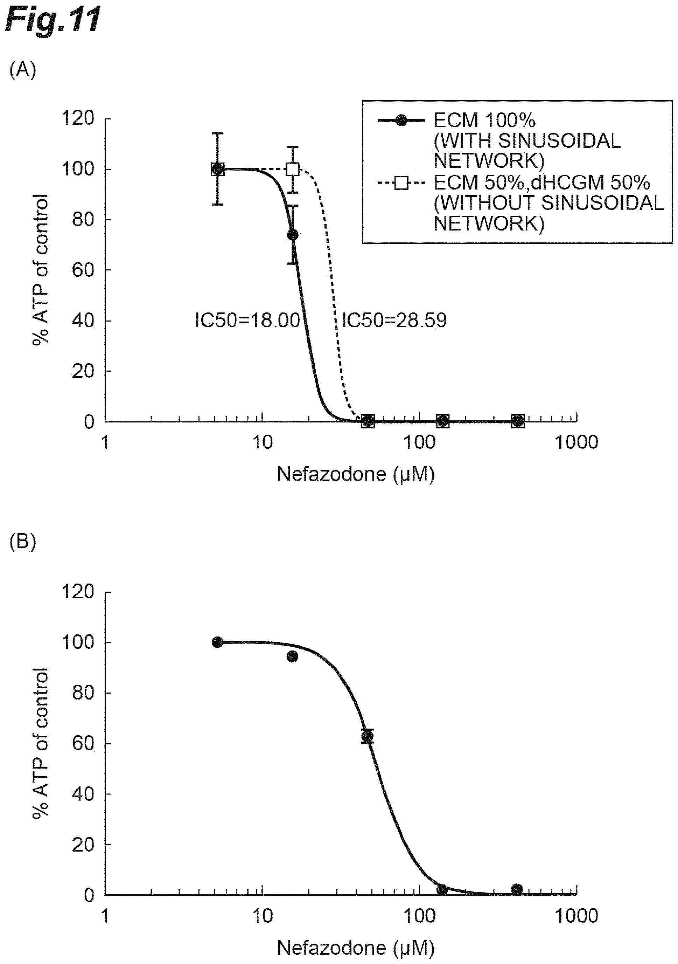

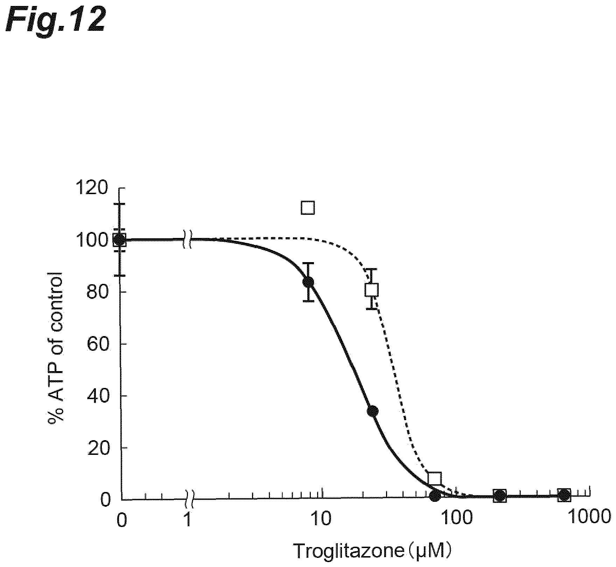

7 days after the start of the culture, the medium was changed to a medium in which the drug (Nefazodone or Troglitazone) was dissolved. 10 days after the start of the culture, again, the medium was replaced with a medium in which the drug was dissolved, and after 14 days, the cell activity was quantified by ATP assay. FIGS. 11 and 12 show the evaluation results of the hepatotoxicity.

It was confirmed that the cell structure having a liver sinusoidal network had better responsiveness to a substance having hepatotoxicity than the cell structure having no liver sinusoidal network.

### <Test Example 7: Monocrotaline administration experiment>

### [Preparation of liver model (hepatotoxicity model)]

### (Recovery of cells other than PXB cells)

The frozen stocks of Lx2, SEC, MSC cells were put to sleep, and cultured based on the protocol recommended by the manufacturer without subculture therebetween, recovery was performed from the culture flask and the petri dish with trypsin based on a general method, and the amount of cells was then measured.

### (Recovery of PXB cells)

The cells were recovered by the following procedure and the amount of cells was measured.

The PXB cells were washed with PBS. 2 mL of 0.25% trypsin-EDTA was added to the PXB cells, and the cells were incubated in the incubator. Then, HCGM was added to the PXB cells, pipetting was performed, the PXB cells were recovered, and measurement was performed with a cell counter.

PXB cells, SEC and LX2 were mixed so that the following proportions and cell amount were obtained, and thereby a cell mixture was obtained.
- Total amount of cells per well: 30,000 cells
- Cell proportions in tissue:
   PXB cells: 65%, SEC: 25%, LX2: 10%

The same amounts of 1.0 mg/mL heparin solution (buffer: 100 mM Tris-HCL) and 0.3 mg/mL collagen solution (buffer: 5 mM acetate) were mixed to obtain a heparin/collagen solution.

100 µL of the heparin/collagen solution was added to the cell mixture and suspended until the cells were visible, and then centrifuged (400 g×2 min), and a viscous component was formed in the solution.

After the supernatant was removed from the solution containing the viscous component, a 20 U/mL thrombin solution (solvent: HCM (culture medium for hepatocytes)) was added so that the final volume of the solution was "the number of wells to be seeded"×2 µL volume of the solution, and thereby a cell suspension was obtained.

10 mg/mL of a fibrinogen solution was put into a 48 plate, and droplets were formed. The cell suspension was added into the droplets, and then left in the incubator for 40 minutes, and a fibrin gel was formed.

0.5 mL of HCM (containing endothelial cell growth supplement) was added to each well in which the fibrin gel was formed, and a liver model was obtained as a cell structure.

### [Administration to liver model]

1 day (Day1) and 4 days (Day4) after the start of the culture of the liver model, administration was performed on the liver model by replacing the medium with a medium containing monocrotaline at a concentration of 2,000 µM, 666 µM, 222 µM, or 74 µM. Here, the compound used was dissolved in advance in DMSO at a high concentration and stored, and during administration, DMSO was contained at a concentration of 1% in the medium. Since 1% DMSO was contained in each administration condition, replacement of the medium with a medium containing only 1% DMSO was performed at the same time, and the result was used as a negative control.

### [ATP assay of liver model]

7 days (Day7) after the start of the culture of the liver model, as described below, an ATP assay using CellTiter-Glo (registered trademark) 3D Cell Viability Assay kit was performed.

The medium containing the compound was removed from each well of the 48 plate, 100 µL of DMEM at room temperature was added, and 100 µL of an ATP assay reagent included in the kit was then added at room temperature. The 48 plate to which the ATP assay reagent was added was shaken with a thermostat shaker for 5 minutes (1,000 rpm, room temperature). After that, the 48 plate was left at room temperature for 25 minutes.

From each well, a total amount of the mixed solution containing a DMEM and an ATP assay reagent was transferred to a 96 plate for light emission measurement, and the light emission intensity was measured with a plate reader.

### [Fixing to liver model, immunostaining and microscopic imaging]

For the samples on which administration was performed, a fixing treatment, a permeation treatment, blocking, a primary antibody treatment, a secondary antibody treatment, imaging and area calculation and evaluation were performed in that order. Hereinafter, the procedure from the fixing treatment to evaluation will be described.

### (Fixing treatment)

6 days (day6) after the start of the culture of the liver model, the 48 plate was removed from the incubator, the medium was removed, washing with PBS was performed, 300 µL of a 4% paraformaldehyde-phosphate buffer (hereinafter referred to as PFA) was then added to each well, and the liver model was fixed. Then, PFA was sufficiently washed and removed.

### (Permeation treatment·blocking)

100 µL of a 0.2 (v/v)% TRITON/1 (w/v)% BSA PBS solution (hereinafter referred to as a BSA solution) was added into the insert of each well, and left at room temperature for 2 hours.

### (Primary antibody treatment)

Mice-derived anti CD31 antibodies were diluted 100-fold with the BSA solution to obtain a primary antibody solution. 100 µL of the primary antibody solution was added into the insert of each well, and left at 4°C for 24 hours. Then, the primary antibody solution was sufficiently washed and removed.

### (Secondary antibody treatment)

Secondary antibodies were diluted 200-fold with the BSA solution to obtain a secondary antibody solution. 100 µL of the secondary antibody solution was added into the insert of each well, and left at room temperature for 1 hour while light was blocked therefrom. Then, the secondary antibody solution was sufficiently washed and removed, and 100 µL of PBS was added to each well.

### (Fluorescence microscopic imaging)

Images were captured with a confocal microscope. Imaging conditions were as follows.
- Lens used: 4x lens
- Imaging mode: non-confocal
- Filter: 647 (Ex)/512 (Em)
- Z-axis position: 0-100 µm/10 µm increment

The intensities of the images captured by the above method were added up to obtain a fluorescence observation image of each liver model.

The captured images were subjected to image analysis using Image J according to the following procedures (1) to (10). Thereby, a framework image extracted from the original image was obtained. The area of the liver sinusoidal network (vessel) in the framework image was extracted by the image cross product.
(1) Image>Type>8-bit
(2) Process>Smooth
(3) Process>Subtract Background>Sliding Paraboloid (20 pixels)
(4) Process>Enhance contrast (Saturated pixels: 10.0%, Normalize)
(5) Process>Math>Subtract (value: 100)
(6) Plugins>Mexican hat filter (Radius: 5.0)
(7) Plugins>Skelton>Skeletonize (2D/3D)
(8) Image>Adjust>Threshold (threshold determination: 1- 255)
(9) Process>Analyze particle (Size: >250 µm)
(10) Analyze>Analyze Skeleton>Analyze Skeleton (2D/3D)

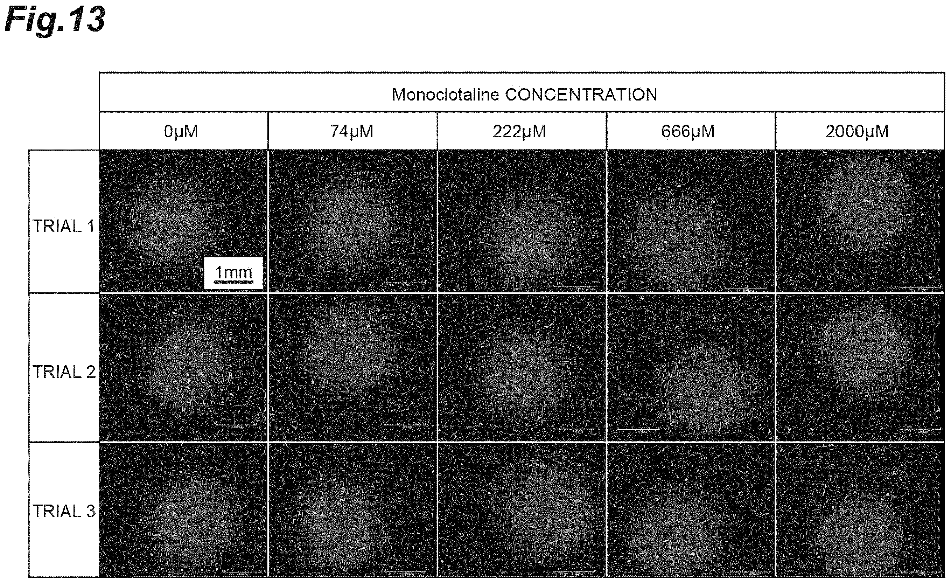

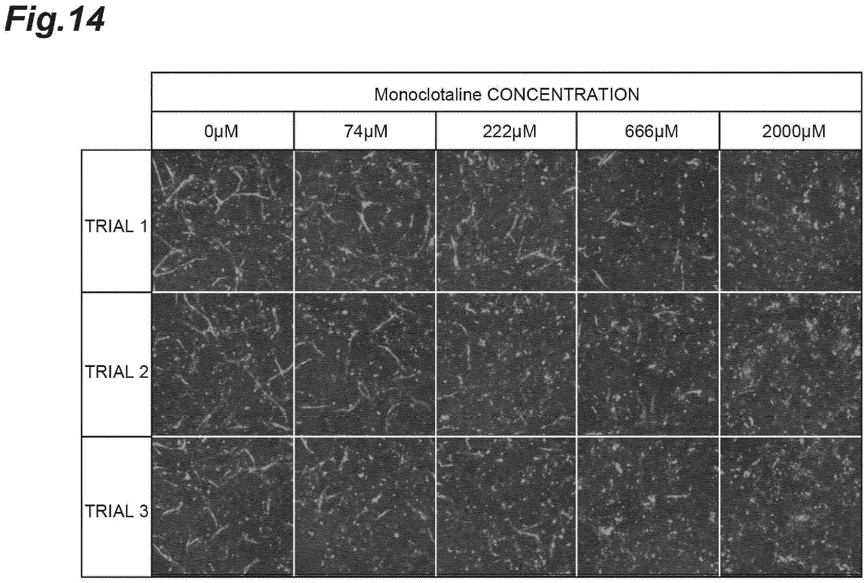

FIG. 13 shows the results of CD31 immunostaining 7 days (Day7) after the start of the culture of the liver model to which monocrotaline with a predetermined concentration was administered. FIG. 14 shows images obtained by extracting a part of 1 m² substantially in the center of the liver model. As shown in FIGS. 13 and 14, it was observed that the liver sinusoidal network became fragmented as the monocrotaline concentration increased.

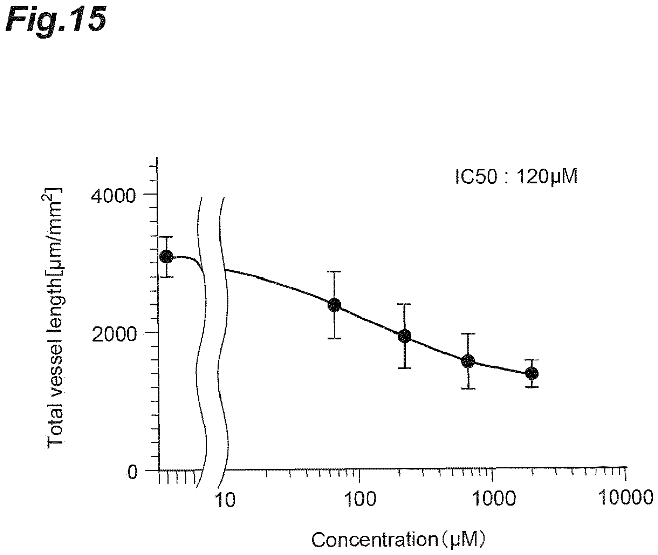

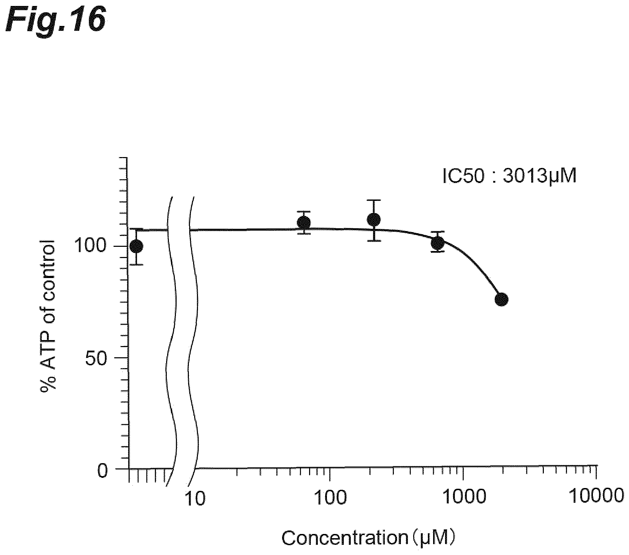

FIG. 15 shows the quantification results of the total vessel length of the liver sinusoidal network. FIG. 16 shows the results of ATP assay. As shown in FIGS. 15 and 16, when the liver sinusoidal network was quantified (when the amount of the vascular network damaged was quantified), the drug efficacy could be evaluated with higher sensitivity than the ATP assay.

### <Test Example 8: Toxicity evaluation experiment of 18 compounds>

The toxicity of the compound was evaluated using a cell structure having a liver sinusoidal network produced using CMF (Example A) and a cell structure having a liver sinusoidal network produced using heparin and a collagen solution (Example B). The toxicity was evaluated by performing administering of the compound and the ATP assay on the compound to be evaluated in the same procedure as in Test Example 6.

The cell structure of Example A was obtained by the method described in Test Example 6.

The cell structure of Example B was obtained by the method described in Test Example 7.

The toxicity was evaluated with IC50 and MOS value. The MOS value is an index used for toxicity evaluation as a value considering the maximum drug concentration (Cmax).

Reference Example A and Reference Example B are literature data based on Proctor WR et al., Arch Toxicol, 2017, 91, 2849-2863 and Supplementary information in the literature. Reference Example A is two-dimensional primary human hepatocytes (2D PHH) in the literature, and Reference Example B is 3D human liver microtissues (3D hLiMT) in the literature. Tables 4 and 5 also show IC50 of Reference Examples A and B. It was shown that the cell structure produced by this test example could be evaluated with favorable reproducibility for those reported to be toxic.

The following tables also show the drug-induced liver injury severity category (DILI severity category, LKBT). In LKBT, III indicates most DILI concern, II indicates less DILI concern, and I indicates no DILI concern.

**[Table 4]**

| | | | | | IC50 [µM] | | | |
|---|---|---|---|---|---|---|---|---|
| No. | Compound name | DILI severity category (LKBT) | Toxicity | Cmax [µM] | Reference Example A | Reference Example B | Example A | Example B |
| 1 | Amiodarone | III | Yes | 5.27 | 45.0 | ∼31.6 | 14.1 | 33.9 |
| 2 | Bosentan | III | | 7.43 | Outside measurement range | ∼93.0 | 67.1 | 209.4 |
| 3 | Nefazodone | III | | 4.26 | 36.2 | ∼29.4 | 22.1 | 32.4 |
| 4 | Perhexiline | III | | 2.16 | 11.2 | ∼2.6 | 5.4 | 3.2 |
| 5 | Tolcapone | III | | 47.58 | 158.1 | ∼19.0 | 32.4 | 88.7 |
| 6 | Troglitazone | III | | 6.39 | Outside measurement range | ∼14.6 | 17.1 | 50.2 |
| 7 | Ximelagatran | III | | 0.45 | Outside measurement range | ∼150.6 | Outside measurement range | Outside measurement range |
| 8 | Acetaminophen | III | | 165.38 | 4596.0 | ∼572.8 | 761.2 | 868.0 |
| 9 | Diclofenac | III | | 10.13 | Outside measurement range | ∼61.4 | 64.1 | 231.7 |
| 10 | Levofloxacin | III | | 15.77 | Outside measurement range | Outside measurement range | Outside measurement range | 960.1 |
| 11 | Nifedipine | II | | 0.29 | Outside measurement range | Outside measurement range | 552.1 | 359.8 |
| 12 | Rosiglitazone | II | | 1.04 | Outside measurement range | 37.7 | 46.5 | 79.1 |
| 13 | Flucloxacillin | II | | 66.10 | 4669.0 | 12176 | 1386.0 | 5800.0 |
| 14 | Cyclophosphamide | II | | 143.00 | Outside measurement range | Outside measurement range | 1671 | 1360 |
| 15 | Minoxidil0 | I | No | 1.19 | Outside measurement range | Outside measurement range | Outside measurement range | Outside measurement range |
| 16 | Flumazenil | I | | 1.12 | Outside measurement range | Outside measurement range | Outside measurement range | Outside measurement range |
| 17 | Phentolamine | I | | 0.09 | Outside measurement range | Outside measurement range | 179.8 | 66.0 |
| 18 | Pyridostigmine | I | | 1.10 | Outside measurement range | Outside measurement range | Outside measurement range | Outside measurement range |

**[Table 5]**

| | | | | | MOS value [IC50/Cmax] | | | |
|---|---|---|---|---|---|---|---|---|
| No. | Compound name | DILI severity category (LKBT) | Toxicity | Cmax [µM] | Reference Example A | Reference Example B | Example A | Example B |
| 1 | Amiodarone | III | Yes | 5.27 | 8.5 | 6.0 | 2.7 | 6.4 |
| 2 | Bosentan | III | | 7.43 | Outside measurement range | 12.5 | 9.0 | 28.2 |
| 3 | Nefazodone | III | | 4.26 | 8.5 | 6.9 | 5.2 | 7.6 |
| 4 | Perhexiline | III | | 2.16 | 5.2 | 1.2 | 2.5 | 1.5 |
| 5 | Tolcapone | III | | 47.58 | 3.3 | 0.4 | 0.7 | 1.9 |
| 6 | Troglitazone | III | | 6.39 | Outside measurement range | 2.3 | 2.7 | 7.9 |
| 7 | Ximelagatran | III | | 0.45 | Outside measurement range | 334.7 | Outside measurement range | Outside measurement range |
| 8 | Acetaminophen | III | | 165.38 | 27.8 | 3.5 | 4.6 | 5.2 |
| 9 | Diclofenac | III | | 10.13 | Outside measurement range | 6.1 | 6.3 | 22.9 |
| 10 | Levofloxacin | III | | 15.77 | Outside measurement range | Outside measurement range | Outside measurement range | 60.9 |
| 11 | Nifedipine | II | | 0.29 | Outside measurement range | Outside measurement range | 1912.1 | 1246.1 |
| 12 | Rosiglitazone | II | | 1.04 | Outside measurement range | 36.1 | 44.6 | 75.8 |
| 13 | Flucloxacillin | II | | 66.10 | 70.6 | 184.2 | 21.0 | 87.7 |
| 14 | Cyclophosphamide | II | | 143.00 | Outside measurement range | Outside measurement range | 11.7 | 9.5 |
| 15 | Minoxidil0 | I | No | 1.19 | Outside measurement range | Outside measurement range | Outside measurement range | Outside measurement range |
| 16 | Flumazenil | I | | 1.12 | Outside measurement range | Outside measurement range | Outside measurement range | Outside measurement range |
| 17 | Phentolamine | I | | 0.09 | Outside measurement range | Outside measurement range | 2090.7 | 766.9 |
| 18 | Pyridostigmine | I | | 1.10 | Outside measurement range | Outside measurement range | Outside measurement range | Outside measurement range |

## Claims

1. A cell structure, comprising:
cells containing at least hepatocytes and vascular endothelial cells, and extracellular matrix components,
wherein the extracellular matrix components are disposed between the cells, and
wherein a liver sinusoidal network is provided between the cells.

2. The cell structure according to claim 1,
wherein the proportion of the number of hepatocytes in the total number of cells is 60% or more and 80% or less.

3. The cell structure according to claim 1 or 2,
wherein the proportion of the number of vascular endothelial cells in the total number of cells is 5% or more and 35% or less.

4. The cell structure according to any one of claims 1 to 3,
wherein the extracellular matrix components are fibrous.

5. The cell structure according to any one of claims 1 to 4,
wherein the extracellular matrix components comprise collagen components.

6. The cell structure according to any one of claims 1 to 5, comprising a polyelectrolyte.

7. The cell structure according to any one of claims 1 to 6,
wherein the extracellular matrix components comprise fragmented extracellular matrix components.

8. The cell structure according to any one of claims 1 to 7,
wherein the vascular endothelial cells are sinusoidal endothelial cells.

9. The cell structure according to any one of claims 1 to 8,
wherein the cells further comprise hepatic stellate cells.

10. A method for evaluating the hepatotoxicity of a substance to be tested, comprising:
a culture step of culturing the cell structure according to any one of claims 1 to 9 in a state of being in contact with a substance to be tested,
wherein the presence or absence or the degree of hepatotoxicity is evaluated using a cell viability, an amount of albumin produced, or an adenosine triphosphate content of the cell structure as an index after the culture step.

11. A method for evaluating the hepatotoxicity of a substance to be tested, comprising
a culture step of culturing the cell structure according to any one of claims 1 to 9 in a state of being in contact with a substance to be tested,
wherein the presence or absence or the degree of hepatotoxicity is evaluated using a liver sinusoidal network in the cell structure as an index after the culture step.

12. The method for evaluating the hepatotoxicity of a substance to be tested according to claim 10 or 11,
wherein the culture step is performed by culturing the cell structure in a medium containing the substance to be tested.

13. A method for producing a cell structure, comprising:
a contact step of contacting cells comprising at least hepatocytes and vascular endothelial cells with extracellular matrix components in an aqueous medium; and
a culture step of culturing the cells in contact with the extracellular matrix components,
wherein the contact step is performed under conditions in which aggregation of the extracellular matrix components in an aqueous medium is restricted, and
wherein the culture step is performed under conditions suitable for culturing hepatic non-parenchymal cells.

14. The method for producing a cell structure according to claim 13, wherein the extracellular matrix components are fibrous.

15. The method for producing a cell structure according to claim 13 or 14,
wherein the contact step is performed by mixing the cells, the extracellular matrix components, and a polyelectrolyte.

16. The method for producing a cell structure according to claim 15, wherein the polyelectrolyte is heparin.

17. The method for producing a cell structure according to any one of claims 13 to 16,
wherein the contact step comprises accumulating the cells and the extracellular matrix components after the cells are brought into contact with the extracellular matrix components.

18. The method for producing a cell structure according to any one of claims 13 to 17,
wherein the extracellular matrix components comprise collagen components.

19. The method for producing a cell structure according to any one of claims 13 to 18,
wherein the extracellular matrix components include fragmented extracellular matrix components.

20. The method for producing a cell structure according to claim 19,
wherein the fragmented extracellular matrix components are defibrated extracellular matrix components.

21. The method for producing a cell structure according to any one of claims 13 to 20,
wherein the vascular endothelial cells are sinusoidal endothelial cells.

22. The method for producing a cell structure according to any one of claims 13 to 21,
wherein the cells further comprise hepatic stellate cells.

23. The method for producing a cell structure according to any one of claims 13 to 22,
wherein the culture step is performed in the presence of angiogenesis promoting factors.
